(19) Europäisches Patentamt
European Patent Office
Office européen des brevets

(11) EP 2 842 982 B1

(12) **EUROPEAN PATENT SPECIFICATION**

(45) Date of publication and mention of the grant of the patent:
**26.04.2017 Bulletin 2017/17**

(21) Application number: **13781759.9**

(22) Date of filing: **25.03.2013**

(51) Int Cl.:
***C08G 59/44*** (2006.01) ***B32B 15/08*** (2006.01)
***B32B 27/38*** (2006.01) ***C07C 233/38*** (2006.01)
***C09J 163/00*** (2006.01)

(86) International application number:
**PCT/JP2013/058636**

(87) International publication number:
**WO 2013/161480 (31.10.2013 Gazette 2013/44)**

(54) **EPOXY RESIN CURING AGENT, EPOXY RESIN COMPOSITION, GAS BARRIER ADHESIVE AGENT, AND GAS BARRIER LAMINATE BODY**

EPOXIDHARZHÄRTUNGSMITTEL, EPOXIDHARZZUSAMMENSETZUNG, GASBARRIEREN-KLEBEMITTEL UND GASBARRIEREN-LAMINATKÖRPER

AGENT DE DURCISSEMENT DE RÉSINE ÉPOXYDE, COMPOSITION DE RÉSINE ÉPOXYDE, AGENT ADHÉSIF PERMÉABLE AUX GAS, ET UN CORPS STRATIFIÉ PERMÉABLE AUX GAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.04.2012 JP 2012103912**
**17.12.2012 JP 2012274973**

(43) Date of publication of application:
**04.03.2015 Bulletin 2015/10**

(73) Proprietor: **Mitsubishi Gas Chemical Company, Inc.**
**Tokyo 100-8324 (JP)**

(72) Inventors:
- **HONDA, Eiichi**
**Hiratsuka-shi**
**Kanagawa 254-0016 (JP)**
- **KOUNO, Kazuki**
**Hiratsuka-shi**
**Kanagawa 254-0016 (JP)**

(74) Representative: **Müller-Boré & Partner Patentanwälte PartG mbB**
**Friedenheimer Brücke 21**
**80639 München (DE)**

(56) References cited:
**WO-A1-2011/115020**
**WO-A1-2011/132637**
**JP-A- 2009 523 876**
**JP-A- 2010 012 708**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to an epoxy resin curing agent, an epoxy resin composition containing the epoxy resin curing agent, and a gas-barrier adhesive containing the epoxy resin composition and a gas-barrier laminate and others.

Epoxy resins have many advantageous properties over other types of resins, such as adhesiveness to various types of base materials, heat resistance, chemical resistance, electrical characteristics and mechanical characteristics. Because of these, epoxy resins have been conventionally used in a wide variety of industrial fields such as coatings used for corrosion proofing or decorative purpose and adhesives for use in engineering works or architecture. Generally, the gas-barrier properties of the epoxy resin compositions used in the fields of coatings or adhesives are excellent compared to compositions using e.g., a urethane resin, an acrylic resin and a polyolefin resin but fall short of those of compositions using materials classified into gas barrier materials such as poly (vinylidene chloride) and polyvinyl alcohol. For the reason, if an epoxy resin is used for this purpose, in order to improve gas-barrier properties, various contrivances have been made, for example, increasing the thickness of a coating film, laminating another material for coating and using a filler in combination.

Meanwhile, methods for improving gas-barrier properties against oxygen and carbon dioxide of coating compositions using an epoxy resin by increasing the content of amine nitrogen in the compositions have been proposed (see Patent Literatures 1 and 2). However, in these coating compositions, the gas-barrier properties thereof is not significantly high and the gas-barrier properties under high-humidity conditions are not excellent. Therefore, further improvement is desired.

Furthermore, a method for improving the gas-barrier properties from those of the above composition and improving the gas-barrier properties under high-humidity conditions has been proposed by using a coating composition in which the ratio of active amine hydrogen in polyamine to epoxy groups in polyepoxide is at least 1.5: 1 and the polyamine serves as an initiation polyamine and is modified polyamine in which at least 50% of carbon atoms are aromatic (see Patent Literature 3). However, the above coating compositions have the following problems. Since a large amount of amino group having unreacted active amine hydrogen remains in a reaction product after application. Accordingly, assuming that the coating composition is applied onto e.g., a metal and concrete for the purpose of anticorrosion and corrosion proofing, excellent performances such as adhesiveness, heat resistance, chemical resistance and electrical characteristics that an epoxy resin inherently has are not expressed. Furthermore, assuming that the coating composition is used as an adhesive for a packaging film for improving gas-barrier properties, performances required for adhesive use, such as adhesiveness and chemical resistance, are not expressed.

Meanwhile, to prevent these problems, as a gas-barrier coating composition having high gas-barrier properties and excellent chemical resistance, an epoxy resin composition containing an epoxy resin and a specific amine based curing agent as a coating-material forming component is proposed (see Patent Literature 4).

By the way, recently, a composite flexible film obtained by combining different types of polymer materials has become mainstream for the reasons of, as a packaging material, strength, product protection, work fitness, advertising effects by printings, or the like. Such a composite film generally comprises a thermoplastic film layer, or the like to become an outer layer having the role of product protection and a thermoplastic film layer, or the like to become a sealant layer. In order to bond these layers together, generally, a dry lamination method by which an adhesive is applied to a laminated film so as to adhere the sealant layer thereto, and an extrusion lamination method by which an anchor coat agent is applied to a laminated film as necessary, and a plastic film to become a molten sealant layer is pressure-bonded and laminated in a film shape are being practiced. Further, in adhesives used in these methods, a two-component polyurethane adhesive comprising a base agent having an active hydrogen group such as a hydroxyl group and a curing agent having an isocyanate group has generally become mainstream in terms of high adhesiveness (for example, Patent Literature 5 and Patent Literature 6).

However, these two-component polyurethane adhesives need to promote curing by aging over a long period of time of one to five days after bonding of films in order to ensure enough adhesiveness since curing reaction thereof is not so fast in general. In addition, if an unreacted isocyanate group remains after curing due to the use of a curing agent having an isocyanate group, the remaining isocyanate group reacts with moisture in the air to produce carbon dioxide, thereby causing a problem of producing bubbles in a laminated film. Meanwhile, as a method for solving these problems, Patent Literature 7 proposes a polyurethane adhesive, and Patent Literature 8 proposes an epoxy-based laminate adhesive.

However, when a packaging material requires gas-barrier properties, since the gas-barrier properties of the above polyurethane adhesives and the epoxy-based adhesive proposed in Patent Literature 8 are not high, various types of gas barrier layers such as a PVDC coat layer, a polyvinyl alcohol (PVA) coat layer, an ethylene-vinyl alcohol copolymer (EVOH) film layer, a metaxylilene adipamide film layer, and an inorganic evaporated film layer in which alumina ($Al_2O_3$), silica ($SiO_2$), or the like is evaporated need to be separately laminated, resulting in disadvantages in the manufacturing cost of a laminated film and an operation process of laminate.

[Patent Literature]

[Patent Literature 1] Japanese examined Patent Publication No. 07-91367
[Patent Literature 2] Japanese examined Patent Publication No. 07-91368
[Patent Literature 3] National Publication of International Patent Application No. 09-511537
[Patent Literature 4] Japanese Patent Laid-Open No. 2002-256208
[Patent Literature 5] Japanese Patent Laid-Open No. 05-51574
[Patent Literature 6] Japanese Patent Laid-Open No. 09-316422
[Patent Literature 7] Japanese Patent Laid-Open No. 2000-154365
[Patent Literature 8] International Publication No. WO 99/60068

However, the coating composition described in Patent Literature 4 above can express relatively excellent gas-barrier properties; however, further improvement has been demanded in recent years. In addition, the gas-barrier coating composition described in Patent Literature 4 above does not have sufficient adhesiveness to various types of plastics, particularly, to polyester, and improvement of adhesiveness is strongly demanded.
The present invention was made in view of the aforementioned problems. An object of the present invention is to provide an epoxy resin curing agent capable of expressing high gas-barrier properties and excellent adhesiveness to various types of plastics, in particular, to polyester.
Another object of the present invention is to provide an epoxy resin composition having not only excellent performances that an epoxy resin has but also high gas-barrier properties and excellent adhesiveness to various types of plastics, in particular, to polyester.
A further object of the present invention is to provide a gas-barrier adhesive having high gas-barrier properties and excellent adhesiveness to various types of plastics, in particular, to polyester.
A still another object of the present invention is to provide a gas-barrier laminate having high gas-barrier properties and excellent adhesiveness to various types of plastics, in particular, to polyester.
The present inventors made intensive studies to solve the above problems, and found that a specific epoxy resin curing agent is capable of expressing high gas-barrier properties and excellent adhesiveness to various types of plastics, in particular, to polyester; not only excellent performances that an epoxy resin has but also high gas-barrier properties and excellent adhesiveness to various types of plastics, in particular, to polyester, can be expressed at the same time by using the epoxy resin curing agent as a curing agent for an epoxy resin composition; and a laminate employing a cured product formed from the epoxy resin composition as a gas barrier layer has high gas-barrier properties and excellent adhesiveness to various types of plastics, in particular, to polyester. Based on the findings, the present invention was accomplished. More specifically, the present invention provides the following 1 to 22 and <1> to <13>.
1. An epoxy resin curing agent characterized by being a reaction product of the following (A) and (B) only:

(A) metaxylylenediamine or paraxylylenediamine;
(B) an unsaturated carboxylic acid represented by the following formula (1) and/or derivatives thereof:

[Formula 1]

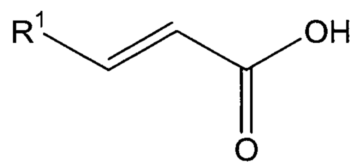

(1)

wherein $R^1$ represents an alkyl group having 1 to 8 carbon atoms, an aralkyl group having 1 to 8 carbon atoms or an aryl group.

2. An epoxy resin curing agent characterized by being a reaction product between the following (A), (B) and further at least one compound selected from the group consisting of the following (C), (D) and (E):

(A) metaxylylenediamine or paraxylylenediamine;

(B) an unsaturated carboxylic acid represented by the following formula (1) and/or derivatives thereof:

[Formula 2]

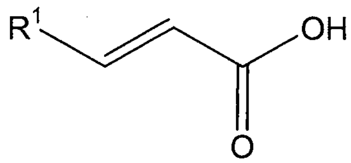

(1)

wherein $R^1$ represents an alkyl group having 1 to 8 carbon atoms, an aralkyl group having 1 to 8 carbon atoms or an aryl group;

(C) monovalent carboxylic acids represented by $R^2$-COOH and/or derivatives thereof, wherein $R^2$ represents a hydrogen atom, an alkyl group having 1 to 7 carbon atoms which may have a hydroxyl group, or an aryl group;

(D) a cyclic carbonate; and

(E) a monoepoxy compound having 2 to 20 carbon atoms.

3. The epoxy resin curing agent according to any one of the above 1 or 2, wherein the (A) component is metaxylylenediamine.

4. The epoxy resin curing agent according to any one of the above 1 to 3, wherein the unsaturated carboxylic acid derivatives of the (B) component represented by the formula (1) are esters, amides, acid anhydrides or acid chlorides.

5. The epoxy resin curing agent according to any one of the above 1 to 4, wherein the (B) component is at least one selected from the group consisting of crotonic acid and crotonic acid esters.

6. The epoxy resin curing agent according to any one of the above 2 to 5, wherein the (C) component is at least one selected from the group consisting of formic acid, acetic acid, propionic acid, butyric acid, lactic acid, glycolic acid, benzoic acid and derivatives of these.

7. The epoxy resin curing agent according to any one of the above 2 to 6, wherein the (D) component is at least one selected from the group consisting of ethylene carbonate, propylene carbonate and glycerin carbonate.

8. The epoxy resin curing agent according to any one of the above 2 to 7, wherein the (E) component is a compound represented by the formula (2):

[Formula 3]

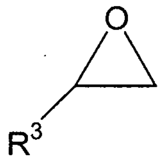

(2)

wherein $R^3$ represents a hydrogen atom, an alkyl group having 1 to 8 carbon atoms, an aryl group, or $R^4$-O-$CH_2$-, and $R^4$ represents a phenyl group or benzyl group.

9. The epoxy resin curing agent according to any one of the above 1 to 8, wherein a reaction molar ratio of the (B) component to the (A) component is in a range of 0.3 to 1.0.

10. An epoxy resin composition at least comprising an epoxy resin and the epoxy resin curing agent according to any one of the above 1 to 9.

11. The epoxy resin composition according to the above 10, wherein a ratio of the number of active amine hydrogen in the epoxy resin curing agent to the number of epoxy groups in the epoxy resin is in a range of 0.2 to 12.0.

12. The epoxy resin composition according to the above 10 or 11, wherein a oxygen permeation coefficient of a cured product obtained by curing the epoxy resin composition is 2.0 ml·mm/$m^2$·day·MPa or less at 23°C and 60%RH.

13. The epoxy resin composition according to any one of the above 10 to 12, wherein the epoxy resin is at least one resin selected from the group consisting of an epoxy resin derived from methaxylylene diamine and having a glycidylamino group, an epoxy resin derived from 1,3-bis(aminomethyl)cyclohexane and having a glycidylamino group, an epoxy resin derived from diaminodiphenylmethane and having a glycidylamino group, an epoxy resin derived from paraaminophenol and having a glycidylamino group and/or a glycidyloxy group, an epoxy resin derived from bisphenol A and having a glycidyloxy group, an epoxy resin derived from bisphenol F and having a glycidyloxy group, an epoxy resin derived from phenolnovolac and having a glycidyloxy group, and an epoxy resin derived from

resorcinol and having a glycidyloxy group.

14. The epoxy resin composition according to the above 13, wherein the epoxy resin comprises the epoxy resin derived from methaxylylene diamine and having a glycidylamino group and/or the epoxy resin derived from bisphenol F and having a glycidyloxy group, as a main component(s).

15. The epoxy resin composition according to the above 14, wherein the epoxy resin contains an epoxy resin derived from metaxylylenediamine and having a glycidylamino group as a main component.

16. A gas-barrier adhesive containing the epoxy resin composition according to any one of the above 10 to 15.

17. A gas-barrier laminate at least comprising a flexible polymer film layer, a paper layer and/or a metal foil layer and at least one gas barrier layer, wherein the gas barrier layer is a layer formed by curing the epoxy resin composition according to any one of the above 10 to 15.

18. The gas-barrier laminate according to the above 17, wherein the gas-barrier layer has the oxygen permeation coefficient of 2.0 ml·mm/m$^2$·day·MPa (23°C, 60%RH) or less.

19. A gas-barrier container obtained by molding a gas-barrier laminate sheet at least having at least one flexible polymer layer and at least one gas barrier adhesive layer, wherein the gas barrier adhesive layer is formed by curing the epoxy resin composition according to any one of the above 10 to 15.

20. A deposited film formed by at least laminating a base material, at least one deposited layer selected from the group consisting of a silica deposited layer, an alumina deposited layer and a silica-alumina two-element deposited layer, a gas-barrier adhesive layer and a sealant layer, wherein the gas barrier adhesive layer is formed by curing the epoxy resin composition according to any one of the above 10 to 15.

21. A gas-barrier laminate obtained by at least laminating a base material, a gas-barrier adhesive layer and a print layer, wherein the gas barrier adhesive layer is formed by curing the epoxy resin composition according to any one of the above 10 to 15.

22. A method for storing a content hermetically by placing the content within a packaging material using a gas-barrier laminate having at least one gas-barrier adhesive layer formed by curing the epoxy resin composition according to any one of the above 10 to 15.

<1> A gas-barrier laminate at least having a flexible polymer film layer, a paper layer and/or a metal foil layer, and at least one barrier layer, wherein the gas barrier layer is a layer formed by curing an epoxy resin composition containing an epoxy resin and an epoxy resin curing agent as main components, and the epoxy resin curing agent is a reaction product of the above (A) and (B) only.

<2> A gas-barrier laminate at least comprising a flexible polymer film layer, a paper layer and/or a metal foil layer, and at least one barrier layer, wherein the gas-barrier layer is a layer formed by curing an epoxy resin composition containing an epoxy resin and an epoxy resin curing agent as main components and the epoxy resin curing agent is a reaction product between the above (A), (B) and further at least one compound selected from the group consisting of the above (C), (D) and (E).

<3> The gas-barrier laminate according to any one of the above <1> or <2>, wherein the (A) component is metaxylylenediamine.

<4> The gas-barrier laminate according to any one of the above <1> to <3>, wherein the unsaturated carboxylic acid derivatives of the (B) component represented by the formula (1) are esters, amides, acid anhydrides or acid chlorides.

<5> The gas-barrier laminate according to any one of the above <1> to <4>, wherein the (B) component is at least one selected from the group consisting of crotonic acid and crotonic acid esters.

<6> The gas-barrier laminate according to any one of the above <2> to <5>, wherein the (C) component is at least one selected from the group consisting of formic acid, acetic acid, propionic acid, butyric acid, lactic acid, glycolic acid, benzoic acid and derivatives of these.

<7> The gas-barrier laminate according to any one of the above <2> to <6>, wherein the (D) component is at least one selected from the group consisting of ethylene carbonate, propylene carbonate and glycerin carbonate.

<8> The gas-barrier laminate according to any one of the above <2> to <7>, wherein the (E) component is a compound represented by the above formula (2).

<9> The gas-barrier laminate according to any one of the above <1> to <8>, wherein a reaction molar ratio [(B)/(A)] of the (B) component to the (A) component is in a range of 0.3 to 1.0.

<10> The gas-barrier laminate according to any one of the above <1> to <9>, wherein the epoxy resin is at least one resin selected from the group consisting of an epoxy resin derived from methaxylylene diamine and having a glycidylamino group, an epoxy resin derived from 1,3-bis(aminomethyl)cyclohexane and having a glycidylamino group, an epoxy resin derived from diaminodiphenylmethane and having a glycidylamino group, an epoxy resin derived from paraaminophenol and having a glycidylamino group and/or a glycidyloxy group, an epoxy resin derived

from bisphenol A and having a glycidyloxy group, an epoxy resin derived from bisphenol F and having a glycidyloxy group, an epoxy resin derived from phenolnovolac and having a glycidyloxy group, and an epoxy resin derived from resorcinol and having a glycidyloxy group.

<11> The gas-barrier laminate according to any one of the above <1> to <10>, wherein the epoxy resin comprises the epoxy resin derived from methaxylylene diamine and having a glycidylamino group and/or the epoxy resin derived from bisphenol F and having a glycidyloxy group, as a main component(s).

<12> The gas-barrier laminate according to any one of the above <1> to <11>, wherein the epoxy resin comprises the epoxy resin derived from methaxylylene diamine having a glycidylamino group, as a main component.

<13> The gas-barrier laminate according to any one of the above <1> to <12>, wherein a gas barrier layer has an oxygen permeation coefficient of 2.0 ml·mm/$m^2$·day·MPa(23°C, 60% RH) or less.

The epoxy resin curing agent and epoxy resin composition of the present invention express high gas-barrier properties and excellent adhesiveness to various types of plastics, in particular, to polyester, at the same time. Therefore, the gas-barrier adhesive containing the epoxy resin composition of the present invention is suitably used as an adhesive to plastic films formed of e.g., polyolefin, polyester and polyamide used in various types of gas permeable base materials, for example, in packaging materials used for food and medicines, and can enhance gas-barrier properties of various types of gas permeable base materials. Furthermore, the epoxy resin composition of the present invention can express excellent performances that an epoxy resin inherently has. Accordingly, the epoxy resin composition can be applied to an object to be coated such as various types of plastic films, plastic containers, metals and concrete to which conventional epoxy resin adhesives are applied.

Furthermore, the gas-barrier laminate of the present invention, which has a gas barrier layer using an epoxy resin composition containing a specific epoxy resin and an epoxy resin curing agent, expresses high gas-barrier properties and excellent adhesiveness to various types of plastics, various materials particularly including polyester, at the same time. Therefore, the gas-barrier laminate of the present invention is preferably used for food, medicines or the like as the purpose of a packaging material.

Now, embodiments of the present invention will be described below. Note that the following embodiments are just examples for explaining the present invention and the present invention is not limited to the embodiments alone.

Now, the epoxy resin curing agent, epoxy resin composition, gas-barrier adhesive and gas-barrier laminate according to embodiments of the invention will be sequentially described, below.

[Epoxy resin curing agent]

The epoxy resin curing agent of the embodiment is a reaction product of the following (A) and (B) only:

(A) metaxylylenediamine or paraxylylenediamine; and

(B) an unsaturated carboxylic acid represented by the formula (1) and/or derivatives thereof:

[Formula 4]

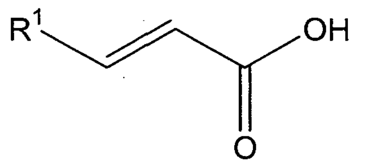

(1)

wherein $R^1$ represents a hydrogen atom, an alkyl group having 1 to 8 carbon atoms, an aralkyl group having 1 to 8 carbon atoms or an aryl group.

The epoxy resin curing agent of the embodiment is improved in gas-barrier properties by using the (A) component, i.e., metaxylylenediamine or paraxylylenediamine, as one of the raw materials thereof. Considering gas-barrier properties, the (A) component is preferably metaxylylenediamine. The (A) components may be used singly or in combination of two or more.

An unsaturated carboxylic acid represented by the above formula (1) and/or derivatives thereof, one of the raw materials serving as the (B) component, is used for the epoxy resin curing agent of the present embodiment in order to express excellent adhesiveness to various types of plastics, in particular, to polyester. Examples of the unsaturated carboxylic acid represented by the above formula (1) and/or derivatives thereof include unsaturated carboxylic acids such as crotonic acid, 2-pentenoic acid, 2-hexenoic acid, 4-methyl-2-pentenoic acid, 2-heptenoic acid, 4-methyl-2-hexenoic acid, 5-methyl-2-hexenoic acid, 4,4-dimethyl-2-pentenoic acid, 4-phenyl-2-butenoic acid, cinnamic acid, o-methyl cinnamic acid, m-methyl cinnamic acid, p-methyl cinnamic acid, 2-octenoic acid, 2-nonenoic acid, 2-decenoic acid and 2-undecenoic acid, and derivatives of these (for example, esters, amides, acid anhydrides, acid chlorides) but not particularly limited to these. The (B) components may be used alone or in combination with two or more types.

Considering higher gas-barrier properties and adhesiveness to polyester, the (B) component is preferably at least one selected from the group consisting of unsaturated carboxylic acids represented by the above formula (1) wherein $R^1$ is a hydrocarbon group having 1 to 3 carbon atoms or a phenyl group, and derivatives thereof; more preferably at least one selected from the group consisting of crotonic acid and crotonic acid derivatives; and still more preferably at least one selected from the group consisting of crotonic acid and crotonic acid esters. As crotonic acid ester, an alkyl ester having 1 to 3 carbon atoms is more preferably, methyl crotonate is still more preferable.

When a carboxylic acid, an ester or an amide is used as the (B) component, the reaction between the (A) component and the (B) component is carried out by blending the (A) component and the (B) component under a condition of preferably 0 to 100°C and more preferably 0 to 70°C, and performing an amide-group-forming reaction under a condition of preferably 100 to 300°C and more preferably 130 to 250°C by e.g., dehydration, dealcoholization and deamination. In this case, during the amide-group-forming reaction, a decompression treatment can be applied to the interior of a reaction apparatus in the final stage of the reaction as necessary in order to complete the reaction. Further, a nonresponsive solvent can be used for dilution as necessary. Furthermore, a catalyst such as phosphites can be added as a dehydrating agent or a dealcoholization agent.

Meanwhile, when the (B) component used is the acid anhydride or the acid chloride, the reaction of the (A) component and the (B) component is carried out by blending the (A) with the (B) under a condition of preferably 0 to 150°C, more preferably 0 to 100°C, and then performing the amide-group-forming reaction. In this case, during the amide-group-forming reaction, the decompression treatment can be applied to the interior of the reaction apparatus in the final stage of the reaction as necessary in order to complete the reaction. Further, the nonresponsive solvent can be used for dilution as necessary. Furthermore, a tertiary amine such as pyridine, picoline, lutidine, and a trialkyl amine can be added.

The amide group site to be introduced by the above amide forming reaction has high cohesion. Owing to the presence of the amide group site in a high ratio in the epoxy resin curing agent, higher oxygen barrier properties and excellent adhesive strength to a base material such as a metal, concrete and plastic are obtained.

Herein, the reaction molar ratio of the (B) component to the (A) component, [(B)/(A)] is not particularly limited; however, the reaction molar ratio is preferably in a range of 0.3 to 1.0. If the above reaction molar ratio is 0.3 or more, a sufficient amount of amide group is produced in an epoxy resin curing agent, with the result that higher level gas-barrier properties and adhesiveness tend to be expressed. In contrast, if the above reaction molar ratio is in a range of 1.0 or less, the amount of amino group required for a reaction (described later) with an epoxy group in the epoxy resin is sufficient, with the result that excellent heat resistance and impact resistance tend to be expressed. Furthermore, solubility to various types of organic solvents or water tends to be increased. In order to enhance gas-barrier properties and coating performance of the epoxy resin cured product to be obtained, the reaction molar ratio of the (B) component to the (A) component, [(B)/(A)] is more preferably in a range of 0.5 to 1.0 and more preferably in a range of 0.6 to 1.0.

The epoxy resin curing agent of the embodiment may be a reaction product of the (A), (B) and further at least one compound selected from the group consisting of (C), (D) and (E) as defined below.

(C) monovalent carboxylic acids represented by $R^2$-COOH and/or derivatives thereof;
wherein $R^2$ represents a hydrogen atom, an alkyl group having 1 to 7 carbon atoms which may have a hydroxyl group, or aryl group;
(D): a cyclic carbonate; and
(E): a monoepoxy compound having 2 to 20 carbon atoms.

Monovalent carboxylic acids represented by $R^2$-COOH and/or derivatives thereof serving as the (C) component is used as necessary in order to reduce reactivity between the epoxy resin curing agent and epoxy resin, thereby improve workability in preparing an epoxy resin composition (as described later). Here, $R^2$ represents a hydrogen atom, an alkyl group having 1 to 7 carbon atoms which may have a hydroxyl group, or an aryl group. Preferably $R^2$ is an alkyl group having 1 to 3 carbon atoms or a phenyl group. Examples of the component (C) include, but not limited to, monovalent carboxylic acid such as formic acid, acetic acid, propionic acid, butyric acid, lactic acid, glycolic acid, and benzoic acid, and derivatives thereof such as an ester, an amide, an acid anhydride, and an acid chloride. The component (C) may be used singly or in combinations of two or more.

The cyclic carbonate serving as the (D) component is used as necessary in order to reduce reactivity between the epoxy resin curing agent and epoxy resin, thereby improve workability in preparing an epoxy resin composition (as described later). The cyclic carbonate serving as the (D) component is preferably a cyclic carbonate having at most six-membered ring, in consideration of the reactivity with the (A) component. Specific examples thereof include ethylene

carbonate, propylene carbonate, glycerin carbonate, 1,2-butylene carbonate, vinylene carbonate, 4-vinyl-1,3-dioxolan-2-one, 4-methoxymethyl-1,3-dioxolan-2-one and 1,3-dioxan-2-one but are not particularly limited to these. Among these, in consideration of gas-barrier properties, at least one selected from the group consisting of ethylene carbonate, propylene carbonate and glycerin carbonate is preferable. The (D) components may be used singly or in combination of two or more types.

The monoepoxy compound serving as the (E) component is a monoepoxy compound having 2 to 20 carbon atoms, which is used as necessary in order to reduce reactivity between the epoxy resin curing agent and epoxy resin, thereby improve workability in preparing an epoxy resin composition (as described later). Considering gas barrier properties, the monoepoxy compound serving as the (E) component is preferably a monoepoxy compound having 2 to 15 carbon atoms and more preferably the compound represented by the following formula (2):

[Formula 5]

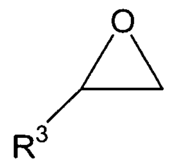

(2)

wherein $R^3$ represents a hydrogen atom, an alkyl group having 1 to 8 carbon atoms, an aryl group, or $R^4$-O-$CH_2$-, and $R^4$ represents a phenyl group or benzyl group.

Examples of the monoepoxy compound represented by the formula (2) include ethylene oxide, propylene oxide, 1,2-butylene oxide, styrene oxide, phenyl glycidyl ether, and benzyl glycidyl ether but not particularly limited to these. The (E) components may be used singly or in combinations of two or more.

In the case where the (C), (D), or (E) components is used, any one of the (C), (D), and (E) components may be used alone or in combination of two or more.

Note that the epoxy resin curing agent of the present embodiment may be a reaction product obtained by a reaction of the (A) and (B) components and the (C) to (E) components used as necessary, to which another component is further added as long as a desired effect is not excessively undermined. As the other component herein, for example, (meth)acrylic acid or a derivative thereof and an aromatic dicarboxylic acid or a derivative thereof are mentioned. Note that the use amount of other component used herein is preferably 30% by mass or less based on the total amount of reaction components constituting the epoxy resin curing agent, more preferably 10% by mass or less and still more preferably 5% by mass or less. Note that in view of gas-barrier properties and adhesiveness to a plastic film, the epoxy resin curing agent of the embodiment is preferably not reacted with components other than the (A) and (B) components. The use amount of components other than the (A) and (B) components is preferably 10% or less by mass based on the total amount of reaction components constituting the epoxy resin curing agent of the embodiment, more preferably 5% by mass or less, and still more preferably 3% by mass or less.

The reaction product of the (A), the (B), and at least one compound selected from the group consisting of (C), (D), and (E) is produced by the reaction of at least one compound selected from the group consisting of (C), (D), and (E) in combination with the component (B), and the component (A) which is a polyamine. In the reaction, the (B), (C), (D) and (E) components are added in no particular order to react with the (A) component, or the (B), (C), (D) and (E) components are blended and collectively react with the (A) component.

The reaction between the (A) component and the (C) component may be performed under the same conditions as in the reaction between the (A) component and (B) component. In the case of using the (C) component, the (B) component and the (C) component may be mixed and collectively reacted with the (A) component or the (A) component and (B) component are first reacted and then reacted with the (C) component.

In the case of using the component (D) and/or the component (E), preferably the reaction of the component (A) and the component (B) is performed prior to the reaction with the component (D) and/or component (E).

The reaction of the component (A), the component (D), and/or the component (E) is performed by mixing the (A), (D), and/or (E) under conditions preferably at 25 to 200°C and forming urethane bonds by addition reaction under conditions preferably at 30 to 180°C, more preferably at 40 to 170°C. At this time, a catalytic agent such as sodium methoxide, sodium ethoxide, and potassium t-buthoxide may be used on an as needed basis. In order to accelerate the urethane bond forming reaction, the (D) and/or (E) may be melted or diluted with an unreactive solvent on an as needed basis.

Also in the case the epoxy resin curing agent of the present embodiment is a reaction product between the (A), the (B) and at least one compound selected from the group consisting of the (C), (D) and (E), the reaction molar ratio of the (B) component to the (A) component, [(B)/(A)], is preferably in a range of 0.3 to 1.0 for the same reason as above,

more preferably in a range of 0.5 to 1.0, and still more preferably in a range of 0.6 to 1.0. In contrast, the reaction molar ratio of (C), (D), and (E) components to the (A) component, [{(C) + (D) + (E) }/(A)], is not particularly limited; however, the ratio is preferably in a range of 0.05 to 3.1, more preferably in a range of 0.07 to 2.5, and still more preferably in a range of 0.1 to 2.0. Considering gas barrier properties and coating properties, the reaction molar ratio of the (B), (C), (D) and (E) components to the (A) component, [{(B) + (C) + (D) + (E)}/(A)], is preferably in a range of 0.35 to 2.5, more preferably in a range of 0.35 to 2.0, and still more preferably in a range of 0.35 to 1.5.

[Epoxy resin composition]

Next, the epoxy resin composition of the embodiment will be described.

The epoxy resin composition having gas-barrier properties of the embodiment (hereinafter, simply referred to as an "epoxy resin composition") contains at least an epoxy resin and the epoxy resin curing agent.

As the epoxy resin, any one of an aliphatic compound, an alicyclic compound, an aromatic compound and a heterocyclic compound having a saturated or unsaturated site may be used. In order to express higher gas-barrier properties, an epoxy resin containing an aromatic ring or an alicyclic structure within a molecule is preferred.

Specific examples of the epoxy resin include an epoxy resin having a glycidylamino group and derived from metaxylylenediamine; an epoxy resin having a glycidylamino group and derived from 1,3-bis(aminomethyl)cyclohexane; an epoxy resin having a glycidylamino group and derived from diaminodiphenyl methane; an epoxy resin having a glycidylamino group and/or glycidyloxy group and derived from paraaminophenol; an epoxy resin having a glycidyloxy group and derived from bisphenol A; an epoxy resin having a glycidyloxy group and derived from bisphenol F; an epoxy resin having a glycidyloxy group and derived from phenol novolak; and an epoxy resin having a glycidyloxy group and derived from resorcinol, but not particularly limited to these. Epoxy resins may be used singly or in combination of two or more types. In order to improve various performances such as flexibility, impact resistance and moist heat resistance, the above various epoxy resins can be blended at a proper rate and used.

Among them, as the epoxy resin used here, a resin comprising an epoxy resin derived from methaxylylene diamine and having a glycidylamino group and/or an epoxy resin derived from bisphenol F and having a glycidyloxy group as a main component is more preferable, and a resin comprising an epoxy resin derived from methaxylylene diamine and having a glycidylamino group as a main component is even more preferable from a viewpoint of the gas-barrier properties. Note that in the specification, "main component" refers to a component contained in an amount of preferably 50 to 100% by mass, more preferably 70 to 100% by mass, and still more preferably 90 to 100% by mass and the epoxy resin used herein may contain components other than the main component(s) within the range.

Various types of epoxy resins as mentioned above can be obtained in accordance with conventional methods. For example, various types of epoxy resins as mentioned above can be each obtained by a reaction between an alcohol, a phenol or an amine and epihalohydrin. For example, the epoxy resin derived from methaxylylene diamine and having a glycidyl amino group is obtainable by adding epichlorohydrin to methaxylylene diamine. At this time, metaxylylenediamine having four amino hydrogens may form mono-, di-, tri- and tetra-glycidyl compounds. The number of substituted glycidyl groups is adjustable by varying the reaction ratio of metaxylylenediamine with epichlorohydrin. For example, an epoxy resin having four glycidyl groups is mainly obtainable by causing addition reaction of four-fold moles of epichlorohydrin to metaxylylenediamine.

More specifically, various types of epoxy resins can be each synthesized by reacting excessive epihalohydrin with an alcohol, a phenol or an amine in the presence of an alkali such as sodium hydroxide, preferably in temperature conditions of 20 to 140°C, and separating the resultant alkali halide. In the case of using an alcohol and a phenol, the reaction temperature is more preferably 50 to 120°C. In the case of an amine, the reaction temperature is more preferably 20 to 70°C.

In the epoxy resin composition of the embodiment, the epoxy resin to be used preferably has a number average molecular weight of 100 to 4,000, more preferably 200 to 1,000, and still more preferably, 200 to 500. The number average molecular weight of the epoxy resin can be controlled, for example, in the above synthesis method, by changing molar ratios of epihalohydrin to an alcohol, a phenol and an amine.

The blending ratio of an epoxy resin and an epoxy resin curing agent in the epoxy resin composition of the embodiment is sufficient if it satisfies a standard blending range generally used in preparing an epoxy resin reaction product (cured product) by the reaction between an epoxy resin and an epoxy resin curing agent and is not particularly limited. Considering gas-barrier properties of the resultant cured product, the ratio of the number of active amine hydrogen in an epoxy resin curing agent to the number of the epoxy groups in the epoxy resin (the number of active amine hydrogen in epoxy resin curing agent/the number of epoxy groups in epoxy resin) is preferably in a range of 0.2 to 12.0, more preferably in a range of 0.4 to 10.0 and still more preferably in a range of 0.6 to 8.0.

Note that the epoxy resin composition of the embodiment may contain a thermosetting resin such as polyurethane-based resin, polyacrylic resin and polyurea-based resin, as necessary.

When the epoxy resin composition of the embodiment is applied to a general base material such as a metal,

concrete and plastic, a wetting agent such as a silicone compound and an acrylic compound may be contained as necessary in order to help moistening of the surfaces of various base materials. Specific examples thereof include BYK331, BYK333, BYK340, BYK347, BYK348, BYK378, BYK380 and BYK381, which are available from BYK-Chemie Japan but are not particularly limited to these. The use amount of wetting agent herein can be appropriately set depending upon performance requirements.

The use amount of wetting agent is preferably in a range of 0.01 to 2.0% by mass based on the total mass of the epoxy resin composition.

Furthermore, the epoxy resin composition of the embodiment may contain a tackifier such as a xylene resin, a terpene resin, a phenol resin and a rosin resin as necessary in order to e.g., improve tackiness to various types of materials. Note that the use amount of tackifier can be appropriately set depending upon performance requirements. The use amount of tackifier is preferably in a range of 0.01 to 2.0% by mass based on the total mass of the epoxy resin composition.

Furthermore, the epoxy resin composition of the embodiment may contain a coupling agent such as a silane coupling agent and a titanium coupling agent as necessary in order to e.g., improve adhesiveness to various types of materials. The use amount of coupling agent herein can be appropriately set depending upon performance requirements. Although it is not particularly limited, the use amount of coupling agent is preferably in a range of 0.01 to 5.0% by mass based on the total mass of the epoxy resin composition.

Furthermore, the epoxy resin composition of the embodiment may contain an inorganic filler such as silica, alumina, mica, talc, aluminum flake and glass flake as necessary in order to e.g., improve various performances such as impact resistance. The use amount of inorganic filler herein can be appropriately set depending upon performance requirements. The use amount of inorganic filler is preferably in a range of 0.01 to 10.0% by mass based on the total mass of the epoxy resin composition.

Furthermore, the epoxy resin composition of the embodiment may contain an anti-foaming agent such as a silicone compound and an acrylic compound as necessary in order to help elimination of foams generated during agitation and blending or coating time. Specific examples of the anti-foaming agent include BYK019, BYK052, BYK065, BYK066N, BYK067N, BYK070 and BYK080, which are available from BYK-Chemie Japan. Among these, BYK065 is more preferable. The use amount of anti-foaming agent herein can be appropriately set depending upon performance requirements. Although it is not particularly limited; the use amount of anti-foaming agent is preferably in a range of 0.01 to 3.0% by mass based on the total mass of the epoxy resin composition.

Furthermore, the epoxy resin composition of the embodiment may contain a compound having an oxygen trapping function as necessary in order to add the oxygen trapping function. Examples of a compound having an oxygen trapping function include low-molecular organic compounds reacting with oxygen such as hindered phenols, vitamin C, vitamin E, an organic phosphorous compound, gallic acid and pyrogallol; and transition metal compounds such as cobalt, manganese, nickel, iron and copper.

Furthermore, the epoxy resin composition of the embodiment may contain various additives known in the art as necessary in order to enhance low-temperature curing properties, add anti-corrosive function or add color. Examples of the additives known in the art include an amine complex of boron trifluoride such as a boron trifluoride-monoethylamine complex; an ether complex of boron trifluoride such as a boron trifluoride-dimethyl ether complex, a boron trifluoride-diethyl ether complex and a boron trifluoride-di-n-butyl ether complex, imidazoles such as 2-phenylimidazole; a catalytic agent for accelerating curing such as a benzoic acid, salicylic acid, dodecyl benzene sulfonic acid, succinic acid, N-ethylmorpholine, dibutyltin dilaurate, cobalt naphthenate and stannous chloride; an organic solvent such as benzyl alcohol, an anti-corrosive agent such as zinc phosphate, iron phosphate, calcium molybdate, vanadium oxide, moisture dispersed silica and fumed silica; an organic pigment such as a phthalocyanine-based organic pigment, and a condensed polycyclic organic pigment; and an inorganic pigment such as titanium oxide, zinc oxide, calcium carbonate, barium sulfate, alumina, and carbon black, but are not particularly limited to these. Use amounts of these may be set as a required amount depending upon the performance requirements.

The curing reaction of the epoxy resin composition of the present embodiment is performed at a concentration of the resin composition and a temperature adequate for producing the curing reaction product. Here, a concentration of the resin composition and a temperature for the curing reaction may be altered depending on the type of the selected material and selection of an application purpose. The temperature for the curing reaction may be generally selected in the range from room temperature to about 140°C. The concentration of the resin composition may be in various states, including the case of using no solvent and the case of diluting the composition to a concentration of about 5 mass% with a proper organic solvent and/or water, depending on the type of the selected material, the molar ratio, and the like. Examples of the proper organic solvent include glycol ethers such as 2-methoxyethanol, 2-ethoxyethanol, 2-propoxyethanol, 2-butoxyethanol, 1-methoxy-2-propanol, 1-ethoxy-2-propanol and 1-propoxy-2-propanol; alcohols such as methanol, ethanol, 1-propanol, 2-propanol, 1-butanol and 2-butanol; non-proton polar solvent such as N,N-dimethylformamide, N,N-dimethylacetamide, dimethylsulphoxide and N-methylpyrrolidone; and water-insoluble solvents such as toluene, xylene, ethyl acetate, ethyl acetate, butyl acetate, acetone and methyl ethyl ketone.

Among these, a water-soluble solvent such as a glycol ether and an alcohol is more preferable.

In the epoxy resin composition of the embodiment, the oxygen permeation coefficient of the cured product obtained by curing the epoxy resin composition is preferably 2.0 ml·mm/m$^2$·day·MPa (23°C, 60% RH) or less, more preferably 1.7 ml·mm/m$^2$·day·MPa (23°C, 60% RH) or less and still more preferably 1.4 ml·mm/m$^2$·day·MPa (23°C, 60% RH) or less.

Note that the epoxy resin composition of the embodiment can be used as a coating material to be applied to an object to be coated such as plastic containers, metals, concrete to which conventional epoxy resin coating materials are applied.

[Gas-barrier adhesive]

The gas-barrier adhesive of the embodiment contains the aforementioned epoxy resin composition of the embodiment. More specifically, the aforementioned epoxy resin composition of the embodiment can be used directly as gas-barrier adhesive or by further blending a solvent other than the aforementioned one, various pigments such as coloring pigments or extender pigments, thereto as necessary.

The gas-barrier adhesive of the embodiment can be applied to metal and concrete in the same manner as an epoxy resin adhesive conventionally used. Furthermore, the gas-barrier adhesive of the embodiment can be used as an adhesive having gas-barrier properties in e.g., packaging materials for medicines requiring high gas-barrier properties, to which conventional epoxy resin adhesives were not able to be applied due to their low gas-barrier properties. In addition, since the gas-barrier adhesive of the embodiment has excellent adhesiveness to a plastic film such as polyolefin, polyester and polyamide, the adhesive can be suitably used as an adhesive for plastic films used as packaging materials for e.g., medicines.

[Laminate]

The gas-barrier laminate of the embodiment is a gas-barrier laminate at least having a flexible polymer film layer (F), a paper layer (P) and/or a metal foil layer (M) and at least one gas barrier layer (G), and at least one gas barrier layer (G) is formed by curing the aforementioned epoxy resin composition of the embodiment as mentioned above. At least one surface of the flexible polymer film layer (F), paper layer (P) and metal foil layer (M) is in a direct contact with the gas barrier layer (G). Further, the gas-barrier laminate of the present embodiment can be used as a gas barrier film on its own, or by further laminating other layers.

The flexible polymer film layer (F), the paper layer (P) and the metal foil layer (M) which constitute the gas-barrier laminate of the present embodiment can be selected arbitrarily depending on the purpose, or can be appropriately combined each other. For example, specific examples of the gas-barrier laminate of the present embodiment include: (F)/(G)/(F), (F)/(G)/(F)/(G)/(F), (F)/(G)/(P)/(G)/(F), (F)/(G)/(P), (F)/(G)/(M)/(G)/(P), (P)/(G)/(M), (P)/(G)/(F)/(G)/(M), (G)/(F)/(G)/(P), (G)/(F)/(G)/(F)/(G), or the like, but is not particularly limited to these. The gas-barrier laminate of the embodiment may further have e.g., an oxygen absorbing layer, an adhesive layer and an anchor coat layer.

By classification based on its role expected, the flexible polymer film layer (F) can function as a base film layer (F1) to hold a gas barrier layer or as a sealant layer (F2) to be a heat seal site in forming a packaging material. Depending upon the role of these layers, performance requirements such as strength and melting point vary.

As the flexible polymer film serving as a base material film layer (F1), any film can be used as long as it can hold a gas barrier layer. Examples thereof include a polyolefin-based film such as low-density polyethylene, high-density polyethylene, linear low-density polyethylene and polypropylene; a polyester-based film such as polyethylene terephthalate and polybutylene terephthalate; a polyamide-based film such as nylon 6, nylon 6,6 and metaxylene adipamide (N-MXD6): a biodegradable film such as a polylactic acid; a polyacrylonitrile-based film; a poly(metha)acrylic-based film; a polystyrene-based film; a polycarbonate-based film; an ethylene-vinyl acetate copolymer saponified material (EVOH)-based film and a polyvinyl alcohol based film. Among them, preferable are the polyolefin-based film, polyester-based film, and polyamide-based film. Further, a film in which coating with various polymers such as a polyvinylidene chloride (PVDC) resin, a polyvinyl alcohol resin, an ethylene-vinyl acetate copolymer suponified material-based resin, or an acrylic resin is applied to these films, a film obtained by depositing various types of inorganic compounds or metal such as silica, alumina, or aluminum to these films, a film obtained by dispersing an inorganic filler or the like to these films, a film to which an oxygen trapping function is imparted to these films, or the like can be also used. Furthermore, the inorganic filler can be dispersed into various types of polymers to be coated. Examples of the inorganic filler include, silica, alumina, mica, talc, aluminum flake, glass flake, but layered silicate such as montmorillonite is preferable. Further, as a dispersion method of the inorganic filler, for example, a conventionally publicly-known method such as an extrusion kneading method, or a mixed dispersion method into a resin solution can be used. Examples of a method for imparting the oxygen trapping function include a method for using at least a part of a low-molecular organic compound which reacts with oxygen such as hindered phenols, vitamin C, vitamin E, an organic phosphorous compound, a gallic acid,

or pyrogallol, and a composition comprising a transition metal compound, such as cobalt, manganese, nickel, iron, or copper.

The thickness of the flexible polymer film serving as a base material film layer (F1) is not particularly limited and appropriately set, preferably 10 μm to 300 μm, more preferably 10 μm to 100 μm, still more preferably 10 μm to 50 μm from a practical viewpoint. Furthermore, the above films may be extended in a uniaxial or biaxial direction.

Note that, in order to form a gas barrier layer with no defects such as film break and eye hole, various surface treatments such as a flame treatment and a corona discharge treatment are desirably applied to the surface of the film material, as necessary. Such surface treatments promote excellent adhesion of the gas barrier layer to the film materials. Further, a print layer can be further provided as necessary after a proper surface treatment has been applied to the surfaces of the film materials. When providing the print layer, general printing facilities which have been used in printing on conventional polymer films such as a photogravure printing machine, a flexo printing machine, and an offset printing machine may be similarly applicable. Furthermore, with regard to an ink forming the print layer, an ink which has been used in a print layer on conventional polymer films containing pigments such as azo and phthalocyanine series, resins such as rosin, a polyamide resin, and polyurethane, and solvents such as methanol, ethyl acetate, and methyl ethyl ketone may be similarly applicable.

With respect to a flexible polymer film serving as a sealant layer (F2), the same film materials exemplified for the base film layer (F1) can be selected. Considering expression of excellent heat-sealing properties, polyolefin-based films such as a polyethylene film, a polypropylene film and an ethylene-vinyl acetate copolymer; films of an ionomer resin, an EAA resin, an EMAA resin, an EMA resin, an EMMA resin and a biodegradable resin are preferable. The thickness of the flexible polymer film serving as a sealant layer (F2) is not particularly limited and can be appropriately set. From a practical viewpoint, the thickness is preferably 10 μm to 300 μm, more preferably 12 μm to 250 μm, and still more preferably 15 μm to 200 μm. Furthermore, to the surface of the film, various surface treatments such as a flame treatment and a corona discharge treatment may be applied.

As a paper layer (P), various publicly-known paper base materials can be used. In forming a paper container, a base material is paper. Therefore, a paper base-material preferably has e.g., shapability, flex resistance, stiffness, elasticity and strength. Specific examples thereof include a bleached or unbleached paper base material; pure white roll paper; craft paper; paperboard; processed paper; recycled paper of these; calcium carbonate paper; and aluminum hydroxide paper. As the above paper base-material, a paper base material preferably having a basis weight of approximately 40 to 600 $g/m^2$ and more preferably having a basis weight of approximately 50 to 500 $g/m^2$ can be suitably used. Note that on the above paper base material, desired printing pictures such as characters, graphics, designs, symbols may be formed by various printing systems.

The metal foil layer (M) and foil of a metal having excellent ductility such as gold, silver, copper, zinc, iron, lead, tin, and alloys of these, steel, stainless steel and aluminum can be used. From an industrial viewpoint, aluminum foil is preferable. The thickness of the metal foil can be appropriately set. Generally, the thickness is preferably 4 to 50 μm.

The surface of a flexible polymer film layer (F), a paper layer (P) or a metal foil layer (M), more specifically, on a surface coated with the epoxy resin composition (adhesive) on which a gas barrier layer (G) to be formed, a primer (medium) layer may be formed. In this case, one-component or two-component primer having various chemical structures can be used as long as the primer has adhesiveness to the base material. Practically, a polyester-based primer having low permeability to an alcohol such as methanol suitably used as a prime solvent of an adhesive is preferable. Furthermore, the thickness of the primer layer is not particularly limited and can be appropriately set. From a practical viewpoint, the thickness is preferably 0.01 μm to 20 μm, more preferably 0.05 μm to 5 μm, and particularly preferably 0.1 μm to 3.0 μm. If the thickness is 0.01 μm to 20 μm, sufficient adhesiveness can be easily expressed and a primer layer having uniform thickness tends to be easily formed.

The gas-barrier laminate of the present embodiment (hereinafter, sometimes referred to as a laminated film) may be a laminate obtained by laminating a thermoplastic resin layer or the like having an outer layer comprising the thermoplastic resin and heat-sealing properties. The gas-barrier laminate of the embodiment may be any gas barrier layer as long as it has at least one adhesive layer containing an epoxy resin and an epoxy resin curing agent as main components and formed by curing the aforementioned epoxy resin composition of the embodiment. Therefore, in the case where a plurality of adhesive layers are present, an adhesive layer(s) other than adhesive layer formed by curing the aforementioned epoxy resin composition of the embodiment may employ an adhesive conventionally known such as a polyurethane based adhesive. Furthermore, resins may be mutually melted to adhere.

Likewise, the laminate film of the embodiment may be any laminate film as long as it contains at least one gas barrier layer formed of a cured product of the aforementioned epoxy resin composition of the embodiment. Other layers can be arbitrarily selected as described above. For example, a laminate film constituted of two-layers such as polyester/a cured product of the aforementioned epoxy resin composition of the embodiment (gas barrier layer), polyolefin/a cured product of the aforementioned epoxy resin composition of the embodiment (gas barrier layer) and polyamide/a cured product of the aforementioned epoxy resin composition of the embodiment (gas barrier layer) and a laminate film constituted of three-layers such as polyolefin/a cured product of the aforementioned epoxy resin composition of the embodiment (gas barrier layer)/polyolefin and polyamide/a cured product of the aforementioned epoxy resin composition of the embodiment (gas barrier layer)/polyolefin are mentioned.

In the gas-barrier laminate of the embodiment, the gas barrier layer (G) is formed by curing the aforementioned epoxy resin composition of the embodiment containing an epoxy resin and epoxy resin curing agent as main components.

[Epoxy cured product]

The epoxy cured product (gas barrier layer (G)) is formed by curing the epoxy resin composition of the above described present embodiment. The oxygen permeation coefficient of the cured product is not particularly limited, preferably 2.0 ml·mm/$m^2$·day·MPa(23°C 60% RH) or less, more preferably 1.7 ml·mm/$m^2$·day·MPa(23°C 60% RH) or less, and even more preferably 1.4 ml·mm/$m^2$·day·MPa(23°C 60% RH) or less.

The curing reaction of an epoxy resin composition for forming a gas barrier layer (G) is performed at the concentration and temperature sufficient to obtain a cured product. Herein, the concentration and curing-reaction temperature of the resin composition may vary depending upon the type of material selected and application purpose. More specifically, the curing-reaction temperature can be selected generally from room temperature to approximately 140°C. The concentration of the resin composition may be in various states, including the case of using no solvent and the case of diluting the composition to a concentration of about 5 mass% with a proper organic solvent and/or water, depending on the type of the selected material or the molar ratio. Examples of the proper organic solvent include, but not limited to, glycol ethers such as 2-methoxyethanol, 2-ethoxyethanol, 2-propoxyethanol, 2-butoxyethanol, 1-methoxy-2-propanol, 1-ethoxy-2-propanol, and 1-propoxy-2-propanol; alcohols such as methanol, ethanol, 1-propanol, 2-propanol, 1-butanol, and 2-butanol; non-proton polar solvent such as N,N-dimethylformamide, N,N-dimethylacetamide, dimethylsulphoxide, and N-methylpyrrolidone; and water-insoluble solvents such as toluene, xylene, methyl acetate, ethyl acetate, butyl acetate, acetone, and methyl ethyl ketone. Among them, water-soluble solvents such as glycol ethers and alcohols are more preferable.

[Production of a laminate]

In the case of laminating various film materials by using the aforementioned epoxy resin composition of the embodiment, a known lamination method such as dry lamination, non-solvent lamination and extrusion lamination can be used. Among these, dry laminate or extrusion laminate is preferable.

In applying the aforementioned epoxy resin composition of the embodiment to various types of materials to laminate various film materials, coating with the epoxy resin composition is carried out at the concentration and temperature sufficient to obtain an epoxy cured product serving as a gas barrier layer (G). The concentration of the resin composition may vary depending upon the type of material selected and the lamination method. More specifically, the concentration of an application liquid of the epoxy resin composition (hereinafter simply referred to also as the "application liquid") may vary from the case of using no solvent to the case of diluting the composition with an appropriate organic solvent and/or water to a concentration of approximately 5% by weight, depending upon the type and molar ratio of material selected and the lamination method. An organic solvent to be used here allows all the solvents having solubility with an epoxy resin composition to be used, and examples thereof include glycol ethers, alcohols, an aprotic polar solvent, a water-insoluble solvent, or the like similar to the above solvents.

The application liquid (an epoxy resin composition) diluted by the above solvents can be diluted at a concentration such that Zahn cup (No. 3) viscosity is within a range of 5 seconds to 30 seconds (25°C) as needed. The concentration of the application liquid can be adjusted appropriately depending on a lamination method. For example, in the dry lamination, the Zahn cup (No. 3) viscosity is preferably 10 seconds to 45 seconds (25°C) during use thereof, and more preferably 10 to 30 seconds.

Furthermore, when a solvent is used the dry temperature of a solvent after application may be sufficient if it is in a range of approximately 20°C to 140°C, and desirable if a temperature which is close to the boiling point of the solvent and has no influence on the object to be coated. For example, in applying to a drawn polypropylene film, the temperature is desirably 40°C to 120°C in consideration of adhesiveness and workability and in order to obtain a laminate film having excellent outer appearance.

As a method for applying the application liquid, a publicly known application system such as roll coating, spray coating, air-knife coating, soaking and brush coating can be used. Among these, roll coating or spray coating is preferable. At this time, for example, a roll coat similar to a case when a polyurethane adhesive component is applied to a polymer film and laminated, or a spray technique and equipment may be applicable.

Furthermore, for example, in using as a primer of a printing machine, general printing facilities used for printing on conventional polymer films, such as a gravure printing machine, a flexo printing machine and an offset printing machine and publicly known coating system such as roll coating, spray coating, air-knife coating, soaking, brush coating and die coating all can be used. Among these, a printing machine or roll coating is preferable. In this case, the same

gravure printing machine or roll coating and facility as in applying gravure ink to a polymer film can be used.

Subsequently, a specific operation in each lamination method will be described.

In the case of the dry lamination method, the application liquid is applied to a film material containing a base material by a roll such as a gravure roll, the solvent is then dried, and to the surface thereof, a new film material is immediately bonded together by a nip roll, thereby making it possible to provide a laminated film. As a solvent for preparing an application liquid, a solvent containing an alcohol having excellent solubility, a comparatively low boiling point, and a carbon number of 3 or less is preferable, and a solvent comprising at least one selected from the group consisting of methanol, ethanol, isopropanol, and n-propanol as a main component is exemplified. Furthermore, a blend liquid obtained by blending a solvent having any functional group of an ester group, ketone group, and aldehyde group which have an effect to delay the reaction of the epoxy resin with the epoxy resin curing agent, suppress thickening of an application liquid, and lengthen operation time is preferable. As the blend liquid obtained by blending the solvent having any functional group of the ester group, ketone group, and aldehyde group, a blend liquid obtained by blending with a solvent having, as a main component, at least one selected from the group consisting of methyl acetate, ethyl acetate, acetone, methyl ethyl ketone, acetaldehyde, and propionaldehyde having a comparatively low boiling point is exemplified. To obtain a laminate film having a low remaining amount of solvent, the content of a solvent having an ester group, a ketone group or an aldehyde group is preferably 20% by weight or less based on the total solvent. Herein, if a solvent remains in a large amount in a laminate film, unpleasant odor is resulted. Thus, the remaining amount of solvent in a laminate film is practically 7 $mg/m^2$ or less. In order to strictly control the odor of a film, the remaining amount of solvent is more preferably 5 $mg/m^2$ or less and still more preferably 3 $mg/m^2$ or less.

In the dry lamination method, an application liquid (epoxy resin composition) can be applied to a sealant layer. For example, the application liquid is applied onto a polyolefin based film such as a polyethylene film, a polypropylene film and an ethylene-vinyl acetate copolymer and dried, and then a base material such as a drawn polypropylene, a polyamide based film, a polyethylene terephthalate film is bonded. In this manner, a laminate film can be produced.

In bonding a film by a nip roll, the films can be bonded by the nip roll kept at 20°C to 120°C. The temperature of the roll is preferably 40 to 100°C. In this case, it is desirable to perform aging of a certain period of time at 20°C to 60°C or the like as necessary after lamination to complete the curing reaction. The epoxy cured product is formed at a sufficient reaction rate by performing the aging of a certain period of time, resulting in the expression of higher gas-barrier properties and adhesion force.

Further, in the case of a non-solvent lamination method, an application liquid previously heated to approximately 40°C to 100°C is applied to a film material including a base material by a roll such as a gravure roll heated to 40°C to 120°C, and then to the surface thereof, a new film material is immediately bonded together, thereby making it possible to provide a laminated film. In this case, it is desirable to perform the aging of a certain period of time as necessary after lamination similarly to the case of the dry lamination method.

In the case of an extrusion lamination method, a diluted solution by an organic solvent of the epoxy resin and the epoxy resin curing agent which are main components of the application liquid as an adhesion adjuvant (anchor coat agent) and/or water is applied to a film material including a base material by the roll such as a gravure roll, drying and curing reaction of the solvent are performed at a temperature of 20°C to 140°C, and then a polymer material melted by an extruder is laminated, thereby making it possible to provide a laminated film. As a polymer material to be melted, a polyolefin-based resin such as a low-density polyethylene resin and linear low-density polyethylene resin, a polypropylene resin, an ethylene-vinyl acetate copolymer resin is preferable.

These lamination methods and other commonly used lamination methods can be combined as necessary, and the layer constitution of the laminated film may be changeable in accordance with the purpose and form.

The thickness of the gas barrier layer can be appropriately set. In order to express higher gas-barrier properties and adhesiveness and form a layer having a uniform thickness, the thickness is preferably in a range of 0.1 to 100 μm, more preferably in a range of 0.3 μm to 20 μm and still more preferably in a range of 0.5 μm to 10 μm.

[Various uses]

The gas-barrier laminate (laminate film) of the embodiment can be used as a packaging material, a packaging bag or a packaging container for protecting food and medicines. In this case, a packaging material may have constituted so as to contain a laminate film in at least part of the gas-barrier laminate. In the case of using the laminate as packaging purpose, the structure of the laminate may vary depending upon the contents, usage environment, and usage form. More specifically, the laminate film (gas-barrier laminate) of the embodiment can be used as it is as a packaging material, and as necessary, an oxygen absorbing layer, a thermoplastic resin layer such as a heat-sealing resin, an optional layer such as a paper layer and/or a metal foil layer can be further laminated. At this time, a layer is formed by using the above application liquid or by using another adhesive and an anchor coating agent. The gas-barrier laminate of the embodiment can be used as a packaging bag or a packaging container by processing it into an arbitrary shape.

(Gas-barrier bag)

Now, a gas-barrier bag such as a soft packaging bag that can be obtained by using the gas-barrier laminate of the embodiment will be described. Such a gas-barrier bag can be produced by using the gas-barrier laminates such that the surfaces of the heat-sealing resin layers of them face each other or the heat-sealing resin layers are superposed and thereafter sealing outer peripheral end portion thereof or superposed portion with heat to form a sealed section. Examples of the form of the sealed section include side seal form, two-side sealed form, three-side sealed form, four-side sealed form, envelope-like sealed form, butt-seam sealed form (pillow-sealed form), pleated sealed form, flat-bottom sealed form, square-bottom sealed form and gazette form.

The gas-barrier bag can be processed into various forms in accordance with contents, usage environment and usage form. Besides, for example, a standing gas-barrier bag (standing pouch) can be formed. Furthermore, for example, a tube form container can be produced by using the above gas-barrier laminate. Here, a heat-seal method can be performed by publicly known methods such as a bar seal, a rotating roll seal, a belt seal, an impulse seal, a high-frequency seal, and an ultrasonic seal. One piece type inlet, two piece type inlet, or other type of inlet, zipper for opening and closing or the like can be optionally attached to the above bag or container.

A packaging product using the above gas-barrier bag can be produced by filling contents in the gas-barrier bag from the opening, and heat-sealing the opening in due time.

(Content to be packaged)

Examples of contents to be packed include confectioneries such as rice confectioneries, bean snacks, nuts, biscuits/cookies, wafer cookies, marshmallows, pies, half-baked cakes, candies, and snack foods; staples such as bread, snack noodle, instant noodle, dry noodle, pasta, aseptic packaging rice, soupy rice, rice gruel, packaging rice cakes, and cereal foods; processed agricultural products such as pickles, cooked beans, natto, fermented soybean paste, frozen bean curd, bean curd, enokidake mushrooms, konjac, processed mountain vegetable products, jams, peanut cream, salads, frozen vegetables, and processed potato products; processed livestock products such as hams, bacons, sausages, processed chicken products, and canned beef; seafood products such as fish hams/sausages, fish jelly products, boiled fish pastes, seaweed, foods boiled in soy sauce, dried bonito, salted fish guts, smoked salmon, and karashi mentaiko; pulps such as peach, mandarin oranges, pineapples, apples, pears, and cherries; vegetables such as corns, asparagus, mushrooms, onions, carrots, white radishes, and potatoes; frozen household dishes such as hamburger steaks, meatballs, seafood fry, dumplings, and croquettes; prepared foods such as chilled ready-made dishes; milk products such as butter, margarine, cheese, cream, instant creamy powder, and modified powder milks for infant; food products such as liquid seasonings, retort curries, and pet foods; cigarettes; disposable body warmers; medicines; cosmetics, and flavor ingredients.

Now, various preferred embodiments having a gas-barrier adhesive layer formed by curing the aforementioned epoxy resin composition of the embodiment will be described, below.

[Gas-barrier container]

The gas-barrier container of the embodiment is a container obtained by molding a gas-barrier laminate sheet at least having at least one flexible polymer layer and at least one gas barrier adhesive layer, and the gas barrier adhesive layer is formed by curing the aforementioned epoxy resin composition of the embodiment.

The flexible polymer layer used herein may be publicly-known film material or sheet material. Specific examples thereof include those mentioned in the above (F), (F1) and (F2) and any further explanation will be omitted herein.

Note that the flexible polymer layer may be e.g., undrawn or uniaxially to biaxially drawn, or a foam product of a polymer. The thickness of the flexible polymer layer is not particularly limited and can be appropriately set. From a practical viewpoint, 0.01 to 5 mm is preferable. Furthermore, in order to form a flexible polymer layer without defects such as film break and eye hole, it is desirable to apply various surface treatments such as a flame treatment and a corona discharge treatment to the surface of the flexible polymer layer, as necessary. Such treatments promote excellent adhesion of the gas barrier adhesive layer to a flexible polymer layer serving as a base material. Furthermore, after a proper surface treatment is applied to the surface of a flexible polymer layer serving as a base material, a print layer can be further provided as necessary. When providing the print layer, general printing facilities which have been used in printing on conventional polymer films such as a photogravure printing machine, a flexo printing machine, and an offset printing machine may be similarly applicable. Furthermore, with regard to an ink forming the print layer, an ink which has been used in a print layer on conventional polymer films containing pigments such as azo and phthalocyanine series, resins such as rosin, a polyamide resin, and polyurethane, and solvents such as methanol, ethyl acetate, and methyl ethyl ketone may be similarly applicable.

Furthermore, on the surface of the flexible polymer layer, more specifically, the coated surface with the epoxy

resin composition (adhesive) for forming a gas barrier adhesive layer, a primer (medium) layer may be formed. In this case, one-component or two-component primer having various chemical structures can be used as long as the primer has adhesiveness with the flexible polymer layer serving as a base material. Practically, a polyester-based primer having low permeability to an alcohol such as methanol suitably used as a prime solvent of an adhesive is preferable. Furthermore, the thickness of the primer layer can be appropriately set. From a practical viewpoint, the thickness is preferably 0.01 μm to 20 μm, more preferably 0.05 μm to 5 μm, and still more preferably 0.1 μm to 3.0 μm. If the thickness is 0.01 μm to 20 μm, sufficient adhesiveness can be easily expressed and a primer layer having uniform thickness tends to be easily formed.

In the gas-barrier container of the embodiment, the gas barrier adhesive layer is formed by curing the aforementioned epoxy resin composition of the embodiment and any further explanation will be omitted herein.

In the gas-barrier container of the embodiment, the thickness of the gas barrier adhesive layer can be appropriately set. From a practical viewpoint, the thickness is preferably 0.1 to 100 μm, and more preferably 0.5 to 10 μm. If the thickness is 0.01 μm to 100 μm, sufficient adhesiveness can be easily expressed and a primer layer having uniform thickness tends to be easily formed.

To the gas-barrier laminate sheet for forming the gas-barrier container of the embodiment, an outer layer formed of a thermoplastic resin and a thermoplastic resin layer having heat-sealing properties may be laminated. The gas-barrier laminate sheet used herein may be any sheet as long as it has at least one gas barrier adhesive layer formed by curing the aforementioned epoxy resin composition of the embodiment. Therefore, in the case where a plurality of adhesive layers are present, an adhesive layer(s) other than the gas barrier adhesive layer formed by curing the aforementioned epoxy resin composition of the embodiment, may employ an adhesive conventionally known such as a polyurethane based adhesive. Furthermore, resins may be mutually melted to adhere.

Likewise, gas-barrier laminate sheet for forming the gas-barrier container of the embodiment may be any sheet as long as it contains at least one of gas barrier adhesive layer formed of a cured product of the aforementioned epoxy resin composition of the embodiment. Other layers can be arbitrarily selected from various types of materials, as described above. For example, a laminate film constituted of two-layers such as polyester/a cured product of the aforementioned epoxy resin composition of the embodiment (gas barrier adhesive layer), polyolefin/a cured product of the aforementioned epoxy resin composition of the embodiment (gas barrier adhesive layer) and polyamide/a cured product of the aforementioned epoxy resin composition of the embodiment (gas barrier adhesive layer); and a laminate film constituted of three-layers such as polyester/a cured product of the aforementioned epoxy resin composition of the embodiment (gas barrier adhesive layer)/polyester, polyester/ a cured product of the aforementioned epoxy resin composition of the embodiment (gas barrier adhesive layer)/polyolefin, polyolefin/a cured product of the aforementioned epoxy resin composition of the embodiment (gas barrier adhesive layer)/polyolefin, and polyamide/a cured product of the aforementioned epoxy resin composition of the embodiment (gas barrier adhesive layer)/polyolefin.

A method for forming the gas-barrier container of the embodiment may be a publicly known method. For example, the gas-barrier container of the embodiment can be obtained by pressurizing and molding the aforementioned gas-barrier laminate sheet into a predetermined shape by a forming method such as press forming, vacuum forming, air-pressure forming, hot-plate forming or plug-assist forming. Furthermore, the gas-barrier container of the embodiment can be obtained by laminating the aforementioned gas-barrier laminate sheet on another flexible film or sheet, and pressurizing and molding it by a forming method such as press forming, vacuum forming, air-pressure forming, hot-plate forming or plug-assist forming into a predetermine shape.

The shape of the gas-barrier container of the embodiment can be appropriately set depending upon performance requirements and use, for example, into a box-shape, a tray shape, a cup shape, a bottle shape and a tube shape. A preferable shape is, for example, defined as follows: Provided that the depth of the container is represented by d and the diameter of the upper surface opening portion of the container is represented by 1, a the ratio of (d/1) of the preferable shape is in a range of 0.01 to 10.0, preferably in a range of 0.02 to 7.0 and more preferably in a range of 0.03 to 5.0. The diameter of the upper surface opening portion of the container herein is defined as the major axis if the shape of the opening portion is an ellipse and defined as the longest diagonal line if the shape is a square, a rectangle or a polygon. According to the gas-barrier container of the embodiment, it is possible to realize a deep-drawn container having the ratio d/l (the ratio of the depth (d) and container upper to the diameter (l) of the surface opening portion of the container) of 1.0 or more and a container having a portion of a large drawn ratio (box-shape container) for example, a corner curve at the bottom having radius (R) of 2 to 5 mm.

The content to be stored in the gas-barrier container of the embodiment is not particularly limited. Specific examples of the content are the same as exemplified in the aforementioned gas-barrier bag. Any further explanation will be omitted herein.

In the gas-barrier container of the embodiment, the gas barrier adhesive layer formed by curing the aforementioned epoxy resin composition of the embodiment has high gas-barrier properties in addition to suitable adhesiveness to various film materials and/or sheet materials and shows high gas-barrier properties in a wide range from low-humidity conditions to high-humidity conditions. Thus, the gas-barrier container of the embodiment expresses extremely high-level gas-barrier properties even if the container does not use a gas-barrier material generally used such as a PVDC coat layer, a polyvinyl alcohol (PVA) coat layer, an ethylene-vinyl alcohol copolymer (EVOH) film layer, a poly metaxylylene adipamide film layer and an inorganic deposited film layer obtained by vapor deposition of alumina or silica. Furthermore, the epoxy resin cured product of the aforementioned embodiment has excellent toughness and humidity/heat resistance. Because of this, a gas-barrier container having excellent impact resistance, resistance to a boiling treatment and resistance to a retort treatment can be also realized.

[Deposited film]

The deposited film of the embodiment is a deposited film obtained by at least laminating a base material, at least one deposited layer selected from the group consisting of a silica deposited layer, an alumina deposited layer and silica-alumina two-element deposited layer, a gas-barrier adhesive layer and a sealant layer; and the gas barrier adhesive layer is formed by curing the aforementioned epoxy resin composition of the embodiment.

As the base material to be used herein, publicly-known film materials or sheet materials may be used. Specific examples include those mentioned in the above (F), (F1) and (F2) and any further explanation will be omitted herein. Furthermore, as the base material to be used herein, materials other than these resin materials can be used. Specifically, films obtained by coating paper sheets such as carton and metal foil such as aluminium and copper, with various polymers such as polyvinylidene chloride (PVDC) resin, a polyvinyl alcohol resin, an ethylene-vinyl acetate copolymer saponified material-based resin and an acrylic resin, can be used. Furthermore, the various polymers used as coating may have an inorganic filler dispersed in the same manner as described in the above (F), (F1) and (F2).

Note that the resin film serving as a base material may be undrawn or uniaxially to biaxially drawn, or a foam product of a polymer. The thickness of the base material is not particularly limited and can be appropriately set. From a practical viewpoint, the thickness is preferably 10 to 300 μm and more preferably 10 to 100 μm. Furthermore, in order to form a flexible polymer layer without defects such as film break and eye hole, it is desirable to apply various surface treatments such as a flame treatment and a corona discharge treatment to the surface of the base material, as necessary. Such treatments promote excellent adhesion of the gas barrier adhesive layer to a base material. Furthermore, after a proper surface treatment is applied to the surface of a base material, a print layer can be further provided as necessary. When providing the print layer, general printing facilities which have been used in printing on conventional polymer films such as a photogravure printing machine, a flexo printing machine, and an offset printing machine may be similarly applicable. Furthermore, with regard to an ink forming the print layer, an ink which has been used in a print layer on conventional polymer films containing pigments such as azo and phthalocyanine series, resins such as rosin, a polyamide resin, and polyurethane, and solvents such as methanol, ethyl acetate, and methyl ethyl ketone may be similarly applicable.

Furthermore, on the surface of the base material, more specifically, the coated surface with the epoxy resin composition (adhesive) for forming a gas barrier adhesive layer, a primer (medium) layer may be formed. In this case, one-component or two-component primer having various chemical structures can be used as long as the primer has adhesiveness with the a base material. From a practical viewpoint, a polyester-based primer having low permeability to alcohol such as methanol, which is suitably used as a main solvent of an adhesive is preferable. Furthermore, the thickness of the primer layer can be appropriately set. From a practical viewpoint, the thickness is preferably 0.01 μm to 20 μm, more preferably 0.05 μm to 5 μm, and particularly preferably 0.1 μm to 3.0 μm. If the thickness is 0.01 μm to 20 μm, sufficient adhesiveness can be easily expressed and a primer layer having uniform thickness tends to be easily formed.

The deposited layer of the deposited film of the embodiment is preferably a layer having transparency and barrier property against oxygen and water vapor. The deposited layer can be formed by vapor deposition of, for example, silica and/or alumina onto the above base material. The deposited layer may be a silica deposited layer or alumina deposited layer, or a silica-alumina two-element deposited layer in which silica and alumina are deposited in two steps. Furthermore, the deposited layer may be formed by forming a thin film of a metal or a metal foil or an inorganic oxide such as silicon, aluminium, magnesium, zinc, tin, nickel or a mixture of these on a base material. Among these, in consideration of resistance to various bactericides, a silica deposited layer, an alumina deposited layer, and a silica-alumina two-element deposited layer are preferable. These vapor deposition methods can be carried out in accordance with conventional methods. For example, various deposited layers can be formed by publicly known various thin film forming processes such as a physical vapor deposition method and a chemical vapor deposition method, more specifically, a vacuum vapor deposition method, a sputtering method and a plasma phase epitaxial method (CVD method).

Note that various deposited films can be used as the above laminate of a base material and a deposited layer. Examples of the deposited film include a silica deposited polyester-based film, a silica deposited polyamide-based film, an alumina deposited polyester-based film, an alumina deposited polyamide-based film, a silica-alumina two-element deposited polyester-based film and a silica-alumina two-element deposited polyamide based film.

Furthermore, in the deposited film of the embodiment, the thickness of the deposited layer can be appropriately

set. From a practical viewpoint, the thickness is preferably 0.1 to 500 nm, more preferably 0.3 to 100 nm, and still more preferably 0.5 to 50 nm. If the thickness is 0.1 to 500 nm, the flex resistance of the deposited film tends to increase.

In the deposited film of the embodiment, the gas barrier adhesive layer is formed by curing the aforementioned epoxy resin composition of the embodiment and any further explanation will be omitted herein.

In the deposited film of the embodiment, the thickness of the gas barrier adhesive can be appropriately set. From a practical viewpoint, the thickness is preferably 0.1 to 100 μm and more preferably 0.5 to 10 μm. If the thickness is 0.01 μm to 100 μm, sufficient adhesion tends to be exerted and a primer layer having uniform thickness tends to be easily formed.

The deposited film of the embodiment herein may have a resin coat layer between the aforementioned deposited layer and the gas-barrier adhesive layer. Furthermore, in laminating individual layers, a conventionally known adhesive such as polyurethane based adhesive and an anchor coating agent may be used. Furthermore, resins may be mutually melted to adhere. In the case of providing an adhesive layer, it is sufficient if the aforementioned gas-barrier adhesive layer is employed as at least one site.

The resin to be employed as a resin coat layer is not particularly limited. Examples thereof include a polyurethane-based resins such as a polyurethane resin, a polyurethane urea resin, an acryl modified urethane resin and an acryl modified urethane urea resin; a vinyl chloride-vinyl acetate copolymer-based resin; rosin-based resins such as rosin modified maleic acid resin; polyamide based resins; polyester-based resins; chlorinated olefin-based resins such as a chlorinated polypropylene resin; a polyethylene imine-based resin; a polybutadiene-based resin and an organic titanium-based resin. A method for forming a resin coat layer is not particularly limited. A resin coat layer is formed, for example, by applying a solution prepared by dissolving the above resin(s) in a solvent such as water, methanol, ethanol, 2-propanol, ethyl acetate, methyl ethyl ketone and toluene, to a deposited layer or a gas-barrier adhesive layer, in accordance with e.g., a gravure method or a roll coat method, followed by drying. At this time, a general printing facility such as a gravure printing machine, a flexo printing machine and an offset printing machine, which has been used for printing on a conventional polymer film, can be also used.

In forming a resin coat layer, the thickness thereof can be appropriately set. From a practical viewpoint, the thickness is preferably 0.005 to 5 μm and more preferably 0.01 to 3 μm. If the thickness is 0.005 to 5 μm, sufficient adhesion tends to be exerted and a resin coat layer having uniform thickness tends to be easily formed.

Note that in using a curable resin as a resin coat layer, either one of one-component and two-component types can be used. In order to improve water resistance and heat resistance, the two-component type is preferred. Furthermore, to add other functions to a resin coat layer, publicly known various additives may be added to the above resins. The additives can be appropriately selected from publicly known ones as necessary and put in use. For example, in order to improve abrasion resistance, blocking prevention, improvement of slip properties and heat resistance and prevention of static charge, wax, a dispersant, an antistatic agent and a surface modifier can be used.

Meanwhile, as the sealant layer in the deposited film of the embodiment, a publicly-known film material or a sheet material can be used and is not particularly limited. Specific examples include those mentioned in the above (F), (F1) and (F2) and any further explanation will be omitted herein. To express excellent heat-sealing properties, the sealant layer is preferably a polyolefin based film such as a polyethylene film, a polypropylene film and an ethylene-vinyl acetate copolymer. The thickness of the sealant layer can be appropriately set. From a practical viewpoint, the thickness is preferably 10 to 300 μm and more preferably 10 to 100 μm. To the surface of the film, various surface treatments such as a flame treatment and a corona discharge treatment may be applied.

The deposited film of the embodiment has excellent laminate strength due to the presence of the gas-barrier adhesive layer formed by curing the aforementioned epoxy resin composition of the embodiment. The laminate strength of the deposited film of the embodiment varies depending upon the quality of a base material and a sealant layer. For example, if the base material is formed of a drawn polypropylene, the laminate strength is preferably 80 g/15 mm or more in the T-peel test performed at a peel speed of 300 mm/min, more preferably 100 g/15 mm or more and still more preferably 120 g/15 mm or more. Conversely, if the base material is formed of drawn nylon or polyethylene terephthalate and the sealant layer is formed of low density polyethylene, the laminate strength is preferably 600 g/15 mm or more in the T-peel test performed at a peel speed of 300 mm/min, more preferably 700 g/15 mm or more and still more preferably 800 g/15 mm or more. In contrast, if the base material is formed of drawn nylon or polyethylene terephthalate, and the sealant layer is formed of an undrawn polypropylene, the laminate strength is preferably 300 g/15 mm or more in the T-peel test performed at a peel speed of 300 mm/min, more preferably 400 g/15 mm or more and still more preferably 500 g/15 mm or more.

On the deposited film of the embodiment, as necessary, e.g., an outer layer consisting of an oxygen absorbing layer or a thermoplastic resin, a thermoplastic resin layer having heat-sealing properties, a paper layer and a metal foil layer may be laminated. The deposited film of the embodiment is any deposited film as long as if it has a gas barrier adhesive layer formed by curing the aforementioned epoxy resin composition of the embodiment.

The deposited film of the embodiment can be used as a packaging material, a packaging bag or packaging container. The same explanation as mentioned above applies to the packaging bag and packaging container and any

further explanation will be omitted herein. Furthermore, the content to be packaged (content) is the same as mentioned above and any further explanation will be omitted herein.

In the deposited film of the embodiment, the gas barrier adhesive layer formed by curing the aforementioned epoxy resin composition of the embodiment has suitable adhesiveness to various film materials and/or sheet materials and high gas-barrier properties, and shows high gas-barrier properties in a wide range from low-humidity conditions to high-humidity conditions. Thus, the deposited film of the embodiment expresses extremely high-level gas-barrier properties even if a gas-barrier material generally used such as a PVDC coat layer, a polyvinyl alcohol (PVA) coat layer, an ethylene-vinyl alcohol copolymer (EVOH) film layer, a poly metaxylylene adipamide film layer or an inorganic deposited film layer obtained by vapor deposition of alumina or silica is not used. Furthermore, the gas barrier adhesive layer formed by curing the aforementioned epoxy resin composition of the embodiment has excellent mechanical strength. Because of this, a deposited film having excellent flex resistance can be also realized.

[Gas-barrier laminate]

The gas-barrier laminate of the embodiment is a gas-barrier laminate formed by at least laminating a base material, a gas-barrier adhesive layer and a print layer, and the gas-barrier adhesive layer is formed by curing the aforementioned epoxy resin composition of the embodiment.

As the base material used herein, a publicly-known film material or sheet material can be used. Specific examples include those mentioned in the above (F), (F1) and (F2) and any further explanation will be omitted herein.

In the gas-barrier laminate of the embodiment, a deposited layer may be present between the above base material and the gas-barrier adhesive layer. The deposited layer is the same as defined in the above deposited film and any further explanation will be omitted herein.

Furthermore, in the gas-barrier laminate of the embodiment, the thickness of the deposited can be appropriately set. From a practical viewpoint, the thickness is preferably 0.1 to 500 nm, more preferably 0.3 to 100 nm and still more preferably 0.5 to 50 nm. If the thickness of the deposited layer is 0.1 to 500 nm, both high gas-barrier properties and formation of uniform thin film tend to be easily obtained. Note that, to improve adhesion between the base material and the deposited layer, an anchor coat layer may be provided between these layers.

In the gas-barrier laminate of the embodiment, the gas barrier adhesive layer is formed by curing the aforementioned epoxy resin composition of the embodiment and any further explanation will be omitted herein.

In the gas-barrier laminate of the embodiment, the thickness of the gas barrier adhesive can be appropriately set. From a practical viewpoint, the thickness is preferably 0.05 to 10 $\mu$m and more preferably 0.1 to 5 $\mu$m. If the thickness of the gas barrier adhesive layer is 0.05 to 10 $\mu$m, both high gas-barrier properties and formation of uniform thin film tend to be easily obtained.

In the gas-barrier laminate of the embodiment, the print layer is a coated film formed of ink containing various ink binder resins, a solvent, various pigments of an azo series and phthalocyanine series, an extender pigment and a stabilizer. The print layer may have patterns of desired printing pictures such as characters, graphics, designs, symbols formed by various printing method. As the ink binder resin used herein, a conventionally known ink binder resin can be appropriately used. Specific examples thereof include a polyurethane-based resins such as a polyurethane resin, a polyester resin, a polyurethane urea resin, an acryl modified urethane resin and an acryl modified urethane urea resin; a vinyl chloride-vinyl acetate copolymer-based resin; rosin-based resins such as rosin modified maleic acid resin; polyamide based resins; chlorinated olefin-based resins such as a chlorinated polypropylene resin; acrylic resins, nitrocellulose-based resins; and rubber-based resins; and are not limited to these. Among these, since it has relatively soft and has adhesiveness, polyurethane-based resin and/or vinyl chloride-vinyl acetate copolymer-based resin are preferred as the ink binder resin. The ink binder resins may be used singly or in combination of two or more types. A print layer can be formed, for example, by dissolving each of these ink binder resins together with various pigments, an extender pigment or a stabilizer in a solvent such as water, methanol, ethanol, 2-propanol, ethyl acetate, propyl acetate, butyl acetate, methyl ethyl ketone and toluene, and applying onto an object to be printed such as a base material in accordance with e.g., a gravure method, a flexo method, an offset method or a roll coat method. When providing the print layer, general printing facilities which have been used in printing on conventional polymer films such as a photogravure printing machine, a flexo printing machine, and an offset printing machine may be similarly applicable. Furthermore, the thickness of the print layer is not particularly limited; however, in consideration of dryness of the ink, generally the thickness is preferably 5 $\mu$m or less.

Note that the ink for forming the print layer, either one-component curing type or two-component curing type can be applicable. When ink of a two-component curing type is used, polyisocyanate is preferably used as a curing agent. Specific examples thereof include aromatic polyisocyanate such as toluene diisocyanate (TDI) and diphenyl methane diisocyanate (MDI); and aliphatic polyisocyanate such as hexamethylene diisocyanate (HMDI), isophorone diisocyanate (IPDI) and xylene diisocyanate (XDI).

Ink for forming the print layer may be, as necessary, diluted to a concentration such that Zahn cup (No. 3)

viscosity thereof is within a range of 5 seconds to 30 seconds (25°C). The concentration of ink may be appropriately controlled in accordance with the lamination method to be used. For example, if a gravure printing machine is used, in order to suppress contamination of a roll and promote formation of a uniform print layer, the Zahn cup (No. 3) viscosity during the use thereof is preferably 10 to 20 seconds (25°C).

The solvent dry temperature after ink coating herein, is not particularly limited; however, it is generally sufficient if the solvent dry temperature is approximately 20 to 140°C and desirable if a temperature which is close to the boiling point of the solvent and has no influence on the object to be coated. For example, in applying ink to a drawn polypropylene film, the temperature is preferably 40°C to 120°C, in consideration of adhesiveness and workability.

To obtain the gas-barrier laminate of the embodiment, the aforementioned epoxy resin composition of the embodiment is applied onto a base material (on a base material or a deposited layer if the deposited layer is formed on the base material) and dried to form a gas barrier adhesive layer, and subsequently, a print layer is formed on the gas barrier adhesive layer. The original sheet of the gas-barrier laminate thus obtained can be rolled up by a roll in accordance with a conventional method. In this case, it is desirable to roll up the original sheet after the print layer is formed thereon. If the original sheet is rolled up by a roller before the print layer is formed, blocking occurs if the aforementioned epoxy resin composition of the embodiment is not sufficiently dried.

Note that, as necessary, various surface treatments (pretreatment) such as a corona discharge treatment, an ozone treatment and a flame treatment can be applied to the surface of the gas-barrier laminate of the embodiment. Furthermore, as necessary, e.g., an oxygen absorbing layer, a base material film layer formed of a thermoplastic resin, a thermoplastic resin layer having heat-sealing properties, a paper layer and a metal foil layer may be laminated on the surface of the gas-barrier laminate of the embodiment. In laminating these layers, the aforementioned epoxy resin composition of the embodiment; a polyester-based, an isocyanate-based (urethane-based), a polyethyleneimine-based, a polybutadiene-based and an organic titanium-based anchor coating agent and the like; and a polyurethane based, a polyacryl-based, a polyester-based, a epoxy-based, a polyvinyl acetate-based, a cellulose-based adhesive and the like for lamination can be used. Furthermore, resins may be mutually melted to adhere. In laminating these layers, publicly known lamination methods such as a wet lamination method, a dry lamination method, a non-solvent type dry lamination method, an extrusion lamination method, a T-die extrusion molding method, a coextrusion lamination method, an inflation method and a coextrusion inflation method can be used. Among these, a dry laminate or an extrusion laminate is preferred.

Specific examples of the base material film layer formed of a thermoplastic resin and a thermoplastic resin layer having heat-sealing properties include those described in the above (F), (F1) and (F2) and any further explanation will be omitted herein.

The gas-barrier laminate of the embodiment can be used as a packaging material, a packaging bag or a packaging container. The explanations of these are the same as described above and any further explanation will be omitted herein.

Furthermore, in the case of producing a paper container for storing liquid containing a paper base material as a gas-barrier container, a laminate sheet is produced by laminating a paper base material on the gas-barrier laminate of the embodiment, as a laminate. From the laminate sheet, a blank plate for forming a desired paper container is produced. Thereafter, using the blank board, a body section, bottom section, a top section and others of a box are formed to produce a paper container for a liquid. In this case, a method for forming a paper container is not particularly limited. For example, publicly known carton shapes such as a brick-type, a flat type or a gable top type may be employed. Furthermore, the shape of the container may be, for example, either a rectangular container or a cylindrical paper such as a round container may be employed.

The gas-barrier laminate of the embodiment, and e.g., a packaging bag or packaging container formed by using this have excellent gas-barrier properties against e.g., oxygen gas and impact resistance, and further has excellent post-processing suitability such as laminate processing, printing processing, and bag and carton formation processing. Thus, these are excellent in packaging suitability and storage suitability of the aforementioned various products.

[Method for storing content]

A gas-barrier laminate having at least one gas-barrier adhesive layer formed by curing the aforementioned epoxy resin composition of the embodiment and various types of laminates, deposited films, packaging bags and packaging containers obtained by thereof are excellent in barrier properties against various flavor components. Therefore, these can be suitably used for packaging purpose for storing various flavor components.

As the contents to be packaged, various types of foods, medicines, toiletry products such as insecticides can be all employed. Furthermore, the contents may be natural substances or synthetic products. Specific examples thereof include confectioneries such as rice confectioneries, bean snacks, nuts, biscuits, cookies, wafer cookies, marshmallows, pies, half-baked cakes, candies, and snack foods; staples such as bread, snack noodle, instant noodle, dry noodle, pasta, aseptic packaging rice, soupy rice, rice gruel, packaging rice cakes, and cereal foods; processed agricultural products such as pickles, cooked beans, natto, fermented soybean paste, frozen bean curd, bean curd, enokidake

mushrooms, konjac, processed mountain vegetable products, jams, peanut cream, salads, frozen vegetables, and processed potato products; processed livestock products such as hams, bacons, sausages, processed chicken products, and canned beef; seafood products such as fish hams/sausages, fish jelly products, boiled fish pastes, seaweed, foods boiled in soy sauce, dried bonito, salted fish guts, smoked salmon, and karashi mentaiko; pulps/citrus such as peach, mandarin oranges, pineapples, apples, pears, cherries, orange grapefruit, lemon and yuzu; vegetables such as corns, asparagus, mushrooms, onions, carrots, white radishes, and potatoes; prepared foods including frozen ready-made dishes and chilled ready-made dishes such as hamburger steaks, meatballs, seafood fry, dumplings, croquettes and retort curries; milk products such as butter, margarine, cheese, cream, instant creamy powder, and modified powder milks for infant; and liquid food such as liquid soup and cooked food; stock sources such as noodle soup, dipping sauce, sauce for rice-bowl cooking, and sukiyaki sauce; liquid foods such as rice vinegar, cereal vinegar, malt vinegar, oligo vinegar, fruit vinegar, cider vinegar, grape vinegar, refresh beverage containing edible vinegar and sweet-sour pork; liquid seasoning such as sushi, vinegared rice, pickling in vinegar, vinegared food, dressing and mayonnaise; foods such as curry, coffee, wasabi, Japanese green tea, vanilla essence, garlic, pet food and cream; spices containing plants of the mint family such as mint, savory, basil, Japanese basil, marjoram, oregano, sage, thyme and rosemary, plants of the solanaceae family such as red pepper and paprika, plants of the Pedaliaceae family such as sesame seed, plants of the composite family such as tarragon, plants of the pepper family such as pepper, plants of the Myristicaceae family such as nutmeg and mace, plants of the Lauraceae family such as Laurel, cinnamon and quassia, plants of the Magnoliaceae family such as star anise, plants of the brassica family such as mustard, wasabi and horseradish, plants of the pea family such as fenugreek, plants of the rue family such as Japanese pepper, plants of the Myrtaceae family such as clove and all spice, plants of the Apiaceae family such as dill, celery, caraway, coriander, cumin, fennel, parsley and anise, plants of the lily family such as garlic and onion, plants of the Iridaceae family such as saffron, plants of the Zingiberaceae family such as ginger, turmeric and cardamom, or plants of the Orchidaceae family such as vanilla beans; medicines such as antiphlogistic analgetic containing methyl salicylate, indomethacin and flufenamic acid; liquid detergent such as shampoo refill, rinse, body soap; toiletry products such as insect repellents, insecticides, disinfectants, sterilizers, hair dyes, deodorant, air freshener and cosmetics.

[Examples]

Now, the present invention is further specifically described by Synthetic Examples and Examples.

A method for evaluating performance of epoxy resin compositions (or adhesive) and laminates according to Examples and Comparative Examples is as follows.

<Oxygen permeation rate (ml/m$^2$·day·MPa)>

Using an oxygen permeation rate measuring device (manufactured by Modern Controls Inc.; OX-TRAN 2/21), the oxygen permeation rate of a laminate film was measured at 23°C under the conditions of a relative humidity of 60%.

<Oxygen permeation coefficient (ml·mm/m$^2$·day·MPa)>

Using the oxygen permeation rate measuring device (manufactured by Modern Controls Inc.; OX-TRAN 2/21), the oxygen permeation rates of the laminate film X, base material and sealant film prepared by the methods described in Examples and Comparative Examples were directly measured at 23°C under conditions of a relative humidity of 60%. Then, the oxygen permeation coefficient of the gas-barrier layer formed of a cured product of the epoxy resin composition (or adhesive) was calculated based on the following formula:

$$1/R_1 = (1/R_2) + (DFT/P) + (1/R_3)$$

In the formula, $R_1$, $R_2$, $R_3$, DFT and P are each defined as follows:

$R_1$ = oxygen permeation rate of laminate film X (ml/m$^2$·day·MPa)
$R_2$ = oxygen permeation rate of the base material (ml/m$^2$·day·MPa)
$R_3$ = oxygen permeation rate of the sealant film (ml/m$^2$·day·MPa)
DFT = thickness of a cured product of an epoxy resin composition (adhesive)(mm)
(note that, in Comparative Example 2, referred to the thickness of a polyurethane adhesive (mm))
P = the oxygen permeation coefficient of a cured product of an epoxy resin composition (adhesive) (ml·mm/m$^2$·day·MPa)

(note that, in Comparative Example 2, referred to the oxygen permeation coefficient of a cured product of polyurethane adhesive)

<Laminate strength (g/15 mm)>

The laminate strength of laminate films Y prepared by the methods described in Examples and Comparative Examples was measured herein by the T-peel test at a peel speed of 300 mm/min, in accordance with the method defined in JIS K-6854.

<Measurement of laminate strength to polyethylene and polyester (g/15 mm)>

The laminate strength of laminate films Z prepared by the methods described in Examples and Comparative Examples was measured herein by the T-peel test at a peel speed of 300 mm/min in accordance with the method defined in JIS K-6854. Note that, in measuring the laminate strength to polyester, to avoid breakage of polyester, which renders it difficult to measure laminate strength, measurement was performed after polyester was reinforced by attaching cellophane tape thereto.

<Oxygen permeation rate (ml/package·day·2.1 MPa) of container>

Using the oxygen permeation rate measuring device (manufactured by Modern Controls Inc.; OX-TRAN 2/21), the oxygen permeation rate (ml/package·day·2.1 MPa) of a container was obtained at 23°C under the conditions of a relative humidity of 60%.

<Outer appearance of molded piece>

Molded pieces were visually observed for wrinkles, insufficient stretching.

Preparation methods of epoxy resin curing agents A to C to be used in Examples and Comparative Examples were as follows.

Synthetic Example 1

(Epoxy resin curing agent A)

A reaction vessel was charged with 1 mol of methaxylylene diamine and 0.93 mol of methyl crotonate. Subsequently, stirring was performed under nitrogen gas stream, at 100°C for 4 hours. While distilling off methanol generated, the temperature was increased to 165°C and maintained at 165°C for 2.5 hours. The corresponding amount of ethanol was added dropwise over 1.5 hours so as to give a solid content concentration of 65%, to obtain an epoxy resin curing agent A.

Synthetic Example 2

(Epoxy resin curing agent B)

A reaction vessel was charged with 0.93 mol of crotonic acid and 4.4 mol of water. Then, after heating to 30°C in nitrogen gas stream, 1 mol of methaxylylene diamine was added dropwise over 1 hour. After heating to 165°C with evaporating off the resultant water, the residue was allowed to stand at 165°C for 2.5 hours. The corresponding amount of ethanol was added dropwise over 1.5 hours so as to give a solid content concentration of 65%, to obtain an epoxy resin curing agent B.

Synthetic Example 3

(Epoxy resin curing agent C)

A reaction vessel was charged with 1 mol of methaxylylene diamine. Then, after heating to 60°C in nitrogen gas stream, 0.93 mol of methyl acrylate was added dropwise over 1 hour. While distilling away methanol generated, the temperature was increased to 165°C and maintained at 165°C for 2.5 hours. Subsequently, corresponding amount of ethanol was added dropwise over 1.5 hours so as to give a solid content concentration of 65%, to obtain an epoxy resin curing agent C.

Example 1

<Preparation of epoxy resin composition A>

First, a solution containing epoxy resin curing agent A (52.5 parts by mass), an epoxy resin (manufactured by Mitsubishi Gas Chemical Company Inc.; TETRAD-X) having a glycidyl amino group and derived from metaxylylenediamine (6.1 parts by mass), ethanol (48.9 parts by mass) and ethyl acetate (7.5 parts by mass) was prepared. To the solution, a silicone anti-foaming agent (manufactured by BYK-Chemie GmbH; BYK065) (0.1 parts by mass) was added and stirred well to obtain an epoxy resin composition A (number of active amine hydrogen in epoxy resin curing agent/number of epoxy groups in an epoxy resin = 3.4).

<Measurement of oxygen permeation coefficient>

Next, the epoxy resin composition A obtained was applied (application quantity: 3.5 g/$m^2$ (solid content)) onto a biaxially drawn polypropylene film (manufactured by TOYOBO CO., LTD.; P2161) of 20 μm in thickness, serving as a base material, by using bar coater No. 8 and dried at 85°C for 10 seconds. Thereafter, a low-density linear polyethylene film (manufactured by Mitsui Chemicals Tohcello Inc.; TUX-MCS) of 40 μm in thickness, serving as a sealant film, was bonded thereto by a nip roll and subjected to aging at 40°C for 2 days. In this manner, laminate film X of Example 1 was obtained.

The oxygen permeation rates of laminate film X, a base material, and sealant film obtained in Example 1 were separately measured under the aforementioned conditions. The oxygen permeation coefficient of the gas-barrier layer formed of a cured product of the epoxy resin composition A was obtained in accordance with the aforementioned formula for computation. The results are shown in Table 1.

<Measurement of laminate strength>

An ethyl acetate solution (solid-content concentration: 30% by mass) containing a polyether component (manufactured by Toyo-Morton, Ltd.; TM-319) (50 parts by mass) and a polyisocyanate component (manufactured by Toyo-Morton, Ltd. CAT-19B)(50 parts by mass) was applied as a polyurethane adhesive onto a biaxially drawn nylon film (manufactured by TOYOBO CO., LTD, N1102) of 15 μm in thickness and dried at 85°C for 10 seconds. Thereafter, a drawn polyester film (manufactured by TOYOBO CO., LTD, trade name: E5200) of 12 μm in thickness was bonded thereto by a nip roll and subjected to aging at 40°C for 2 days to obtain a laminate film serving as a base material.

Using bar coater No. 8, the epoxy resin composition A was applied (application quantity: 3.5 g/$m^2$ (solid content)) onto the polyester film of the obtained laminate film and dried at 85°C for 10 seconds. Thereafter, a low-density linear polyethylene film (manufactured by Mitsui Chemicals Tohcello Inc., trade name: TUX-MCS) of 40 μm in thickness was bonded thereto by a nip roll and subjected to aging at 40°C for 2 days to obtain the laminate film Y of Example 1.

The laminate strength of the laminate film Y of Example 1 was measured by the aforementioned method. The results are shown in Table 1.

<Measurement of laminate strength to polyethylene and polyester>

Using bar coater No. 8, the epoxy resin composition A obtained was applied (application quantity: 3.5 g/$m^2$ (solid content)) onto a drawn polyester film (manufactured by TOYOBO CO., LTD, trade name: E5200) of 12 μm in thickness, serving as a base material, and dried at 85°C for 10 seconds. Thereafter, a low-density linear polyethylene film (manufactured by Mitsui Chemicals Tohcello Inc., trade name: TUX-MCS) of 40 μm in thickness, serving as a sealant film, was bonded thereto by a nip roll and subjected to aging at 40°C for 2 days to obtain the laminate film Z of Example 1.

Using laminate film Z of Example 1, the laminate strength between polyethylene and the adhesive and the laminate strength between polyester and the adhesive were measured by the aforementioned method. The results are shown in Table 2.

Example 2

<Preparation of epoxy resin composition B>

First, a solution containing epoxy resin curing agent B (52.5 parts by mass), an epoxy resin (manufactured by Mitsubishi Gas Chemical Company Inc.; TETRAD-X) having a glycidyl amino group and derived from metaxylylenediamine (6.1 parts by mass), ethanol (48.9 parts by mass) and ethyl acetate (7.5 parts by mass) was prepared. To the solution, a silicone anti-foaming agent (manufactured by BYK-Chemie GmbH; BYK065) (0.1 parts by mass) was added

and stirred well to obtain an epoxy resin composition B (number of active amine hydrogen in epoxy resin curing agent/number of epoxy groups in an epoxy resin = 3.4).

Next, laminate film X of Example 2 was obtained in the same manner as in Example 1 except that the epoxy resin composition B was used in place of the epoxy resin composition A.

<Measurement of oxygen permeation coefficient>

The oxygen permeation coefficient of a gas-barrier layer formed of a cured product of the epoxy resin composition B was measured by the same manner as in Example 1. The results are shown in Table 1.

<Measurement of laminate strength>

Laminate film Y of Example 2 was obtained in the same manner as in Example 1 except that the epoxy resin composition B was used in place of the epoxy resin composition A.

The laminate strength of laminate film Y of Example 2 was measured by using the aforementioned method. The results are shown in Table 1.

<Measurement of laminate strength to polyethylene and polyester>

Laminate film Z of Example 2 was obtained in the same manner as in Example 1 except that the epoxy resin composition B was used in place of the epoxy resin composition A.

Using laminate film Z of Example 2, the laminate strength between polyethylene and the adhesive and the laminate strength between polyester and the adhesive were measured by the aforementioned method. The results are shown in Table 2.

Example 3

Laminate film X of Example 3 was obtained in the same manner as in Example 1 except that the epoxy resin composition B was used in place of the epoxy resin composition A.

<Measurement of oxygen permeation coefficient>

The oxygen permeation coefficient of a gas-barrier layer formed of a cured product of the epoxy resin composition B was measured by the same manner as in Example 2. The results are shown in Table 1.

<Measurement of laminate strength>

The epoxy resin composition B prepared in Example 2 was applied (application quantity: 3.5 g/$m^2$ (solid content)) by using bar coater No. 8 onto a biaxially drawn nylon film (manufactured by TOYOBO CO., LTD, N1102) of 15 μm in thickness, dried at 85°C for 10 seconds. Thereafter, a low-density linear polyethylene film (manufactured by Mitsui Chemicals Tohcello Inc., trade name: TUX-MCS) of 40 μm in thickness was bonded thereto by a nip roll and subjected to aging at 40°C for 2 days to obtain the laminate film Y of Example 3.

The laminate strength of the laminate film Y of Example 3 was measured by the aforementioned method. The results are shown in Table 1.

Example 4

Laminate film X of Example 3 was obtained in the same manner as in Example 1 except that the epoxy resin composition B was used in place of the epoxy resin composition A.

<Measurement of oxygen permeation coefficient>

The oxygen permeation coefficient of a gas-barrier layer formed of a cured product of the epoxy resin composition B was measured by the same manner as in Example 2. The results are shown in Table 1.

<Measurement of laminate strength>

Laminate film Y of Example 4 was obtained in the same manner as in Example 3 except that a biaxially drawn

polypropylene film (manufactured by TOYOBO CO., LTD, Pylen film P2161) of 20 μm in thickness was used in place of the biaxially drawn nylon film (manufactured by TOYOBO CO., LTD, N1102) of 15 μm in thickness.

The laminate strength of the laminate film Y of Example 4 was measured by the aforementioned method. The results are shown in Table 1.

Comparative Example 1

<Preparation of epoxy resin composition C>

First, a solution containing epoxy resin curing agent C (51.9 parts by mass), an epoxy resin (manufactured by Mitsubishi Gas Chemical Company Inc.; TETRAD-X) having a glycidyl amino group and derived from metaxylylenediamine (6.4 parts by mass), ethanol (49.1 parts by mass) and ethyl acetate (7.5 parts by mass) was prepared. To the solution, a silicone anti-foaming agent (manufactured by BYK-Chemie GmbH; BYK065) (0.1 parts by mass) was added and stirred well to obtain an epoxy resin composition C (number of active amine hydrogen in epoxy resin curing agent/number of epoxy groups in an epoxy resin = 3.4).

Next, laminate film X of Comparative Example 1 was obtained in the same manner as in Example 1 except that the epoxy resin composition C was used in place of the epoxy resin composition A.

<Measurement of oxygen permeation coefficient>

The oxygen permeation coefficient of a gas-barrier layer formed of a cured product of the epoxy resin composition C was measured by the same manner as in Example 1. The results are shown in Table 1.

<Measurement of laminate strength>

Laminate film Y of Comparative Example 1 was obtained in the same manner as in Example 1 except that the epoxy resin composition C was used in place of the epoxy resin composition A.

The laminate strength of laminate film Y of Comparative Example 1 was measured by using the aforementioned method. The results are shown in Table 1.

<Measurement of laminate strength to polyethylene and polyester>

Laminate film Z of Comparative Example 1 was obtained in the same manner as in Example 1 except that the epoxy resin composition C was used in place of the epoxy resin composition A.

Using laminate film Z of Comparative Example 1, the laminate strength between polyethylene and the adhesive and the laminate strength between polyester and the adhesive were measured by the aforementioned method. The results are shown in Table 2.

Comparative Example 2

Laminate film of Comparative Example 2 was obtained in the same manner as in Example 1 except that as a polyurethane based adhesive, an ethyl acetate solution (solid content concentration: 30% by mass) containing a polyether component (manufactured by Toyo-Morton, Ltd.; TM-319) (50 parts by mass) and a polyisocyanate component (manufactured by Toyo-Morton, Ltd.; CAT-19B)(50 parts by mass) was used in place of the epoxy resin composition A.

<Measurement of oxygen permeation coefficient>

The oxygen permeation coefficient of an adhesive layer formed of a cured product of a polyurethane based adhesive was measured by the same manner as in Example 1. The results are shown in Table 1.

<Measurement of laminate strength>

Laminate film Y of Comparative Example 2 was obtained in the same manner as in Example 1 except that a polyurethane based adhesive was used in place of the epoxy resin composition A.

The laminate strength of laminate film Y of Comparative Example 2 was measured by using the aforementioned method. The results are shown in Table 1.

<Measurement of laminate strength to polyethylene and polyester>

Laminate film Z of Comparative Example 2 was obtained in the same manner as in Example 1 except that a polyurethane based adhesive was used in place of the epoxy resin composition A.

Using laminate film Z of Comparative Example 2, the laminate strength between polyethylene and the adhesive and the laminate strength between polyester and the adhesive were measured by the aforementioned method. The results are shown in Table 2.

[Table 1]

| | Oxygen permeation coefficient of gas barrier layer (ml·mm/$m^2$·day. MPa) | Oxygen permeation rate of laminate (ml/$m^2$·day. MPa) | Laminate strength (g/15 mm) |
|---|---|---|---|
| Example 1 | 0.89 | 140 | 230 |
| Example 2 | 0.93 | 143 | 220 |
| Example 3 | 0.93 | 167 | 700 |
| Example 4 | 0.93 | 266 | 110 |
| Comparative Example 1 | 0.41 | 85 | 40 |
| Comparative Example 2 | >5.0 | 310 | 300 |

[Table 2]

| | Laminate strength to polyester (g/15 mm) | Laminate strength to polyethylene (g/15 mm) |
|---|---|---|
| Example 1 | 230 | 750 |
| Example 2 | 220 | 770 |
| Comparative Example 1 | 40 | 730 |
| Comparative Example 2 | 300 | not measured |

As shown in Table 1, the cured products of the epoxy resin compositions (gas-barrier adhesives) containing the epoxy resin curing agent of the present invention had high gas-barrier properties and laminate films prepared by using the adhesives all showed high laminate strength. In contrast, the epoxy resin composition of Comparative Example 1 using epoxy resin curing agent C using methyl acrylate in place of the (B) component of the present invention had excellent gas-barrier properties but resulted in significantly poor laminate strength. Furthermore, in Comparative Example 2 using a conventionally known polyurethane based adhesive in place of a gas-barrier adhesive formed of the epoxy resin composition of the present invention, laminate strength was excellent but sufficient gas-barrier properties were not obtained.

Furthermore, as shown in Table 1, the gas-barrier laminates of the present invention using the epoxy resin composition of the present invention had high gas-barrier properties and the laminates (laminate films) of the present invention all showed high laminate strength. In contrast, the laminate of Comparative Example 1 using methyl acrylate in place of the (B) component of the present invention had excellent gas-barrier properties but resulted in significantly poor laminate strength. Furthermore, in the laminate of Comparative Example 2 using a conventionally known polyurethane adhesive in place of the gas-barrier adhesive constituted of the epoxy resin composition of the present invention, laminate strength was excellent but sufficient gas-barrier properties were not obtained.

Furthermore, as shown in Table 2, the laminate film prepared by using the epoxy resin composition (gas barrier adhesive) containing the epoxy resin curing agent of the present invention expressed high laminate strength to either one of polyester and polyethylene.

In contrast, the epoxy resin composition of Comparative Example 1 using epoxy resin curing agent C containing methyl acrylate in place of the (B) component of the present invention resulted in significantly poor laminate strength to polyester. Furthermore, in the laminate of Comparative Example 2 using a conventionally known polyurethane adhesive in place of the gas-barrier adhesive of the epoxy resin composition of the present invention, laminate strength was excellent but sufficient gas-barrier properties were not obtained, as shown in Table 1.

Example 5

On an undrawn amorphous polyester sheet (manufactured by Mitsubishi Chemical Corporation, trade name: Novaclear SG007) of 250 μm in thickness serving as a base material, the above epoxy resin A was applied by a gravure roll (100 lines/cm, depth: 100 μm), dried in a dry oven having a temperature of 60°C (near inlet) to 90°C (near outlet). Thereafter, an undrawn amorphous polyester sheet (manufactured by Mitsubishi Chemical Corporation, trade name: Novaclear SG007) of 250 μm in thickness serving as a bonding material was bonded thereto by a nip roll heated to 50°C and rolled up at a roll-up speed of 40 m/min. The roll was subjected to aging at 40°C for 2 days to obtain laminate sheet X.

<Measurement of oxygen permeation coefficient>

The oxygen permeation rates of laminate sheet X, base material and bonding material of Example 5 were separately measured under the aforementioned conditions. The oxygen permeation coefficient of the gas barrier adhesive layer formed of a cured product of the epoxy resin composition A. The results are shown in Table 3.

<Measurement of laminate strength>

Laminate sheet Y of Example 5 was prepared in the same conditions as those used in forming laminate sheet X of Example 5. The laminate strength of laminate sheet Y was measured by the aforementioned method. The results are shown in Table 3.

<Outer appearance and oxygen permeation rate (ml/package·day·2.1 MPa) of molded piece>

Using laminate sheet X of Example 5, a box shape container of Example 5 having a depth of 2.5 cm, an opening portion having an opening major axis of 7.9 cm and a bottom of 6.3 cm-square was prepared by vacuum molding. The obtained container was evaluated for outer appearance and the oxygen permeation rate of the container was measured. The results are shown in Table 3.

Example 6

The same procedure as in Example 5 was repeated except that the epoxy resin composition B were used in place of the epoxy resin compositions A to prepare laminate sheets X and Y of Example 6 and a box shape container.

The oxygen permeation coefficient of the gas barrier adhesive layer formed of a cured product of the epoxy resin composition B, the laminate strength of laminate sheet Y, the outer appearance of the container and the oxygen permeation rate of the container were obtained in the same manner as in Example 5. The results are shown in Table 3.

Example 7

The same procedure as in Example 5 was repeated except that an undrawn amorphous polypropylene film (manufactured by TOYOBO CO, LTD, trade name: Pylen film P1128) of 40 μm in thickness was used as a bonding material in place of the undrawn amorphous polyester sheet (manufactured by Mitsubishi Chemical Corporation, trade name: Novaclear SG007) of 250 μm in thickness to prepare laminate sheets X and Y and a box shape container of Example 7.

The oxygen permeation coefficients of the gas barrier adhesive layer formed of a cured product of the epoxy resin composition A, the laminate strength of laminate sheet Y, the outer appearance of the container, the oxygen permeation rate of the container were obtained in the same manner as in Example 5. The results are shown in Table 3.

Example 8

The same procedure as in Example 7 was repeated except that the epoxy resin composition B were used in place of the epoxy resin composition A to prepare laminate sheets X and Y and a box shape container of Example 8.

The oxygen permeation coefficients of the gas barrier adhesive layer formed of a cured product of the epoxy resin composition B, the laminate strength of laminate sheet Y, the outer appearance of the container, and the oxygen permeation rate of the container were obtained in the same manner as in Example 5. The results are shown in Table 3.

[Table 3]

| | Gas barrier adhesive layer | | Container | |
|---|---|---|---|---|
| | Oxygen permeation coefficient (ml·mm/$m^2$·day. MPa) | Laminate strength (g/15 mm) | Outer appearance | Oxygen permeation rate (ml/pac·day· 2.1 MPa) |
| Example 5 | 0.89 | 850 | Excellent | 0.020 |
| Example 6 | 0.93 | 830 | Excellent | 0.018 |
| Example 7 | 0.89 | 900 | Excellent | 0.035 |
| Example 8 | 0.93 | 920 | Excellent | 0.037 |

Example 9

To the silica deposited surface of a deposited film (manufactured by Mitsubishi Plastics, Inc. trade name: TECHBARRIER L) prepared by depositing silica onto a polyethylene terephthalate film of 12 μm in thickness, the above epoxy resin composition A was applied by a gravure roll (100 lines/inch, depth; 100 μm), dried in a dry oven having a temperature of 60°C (near inlet) to 90°C (near outlet). Thereafter, a drawn polyester film (manufactured by TOYOBO CO, LTD, trade name: E5200) of 12 μm in thickness was bonded thereto by a nip roll heated to 70°C, rolled up at a roll-up speed of 130 m/min. The roll was subjected to aging at 40°C for 2 days to obtain a laminate film consisting of a base material/deposited layer/gas-barrier adhesive layer/polyester layer.

Subsequently, to the polyester layer surface of the obtained laminate film, the above epoxy resin composition A was applied by a gravure roll (100 lines/inch, depth 100 μm) and subsequently dried in a dry oven having a temperature of 60°C (near inlet) to 90°C (near outlet). Thereafter, a low-density linear polyethylene film (manufactured by Mitsui Chemicals Tohcello Inc., trade name: TUX-MCS) of 40 μm in thickness was bonded thereto by a nip roll heated to 70°C, rolled up at a roll-up speed of 130 m/min. The roll was subjected to aging at 40°C for 2 days to obtain laminate film X of Example 9 consisting of base material/deposited layer/gas-barrier adhesive layer/polyester layer/gas-barrier adhesive layer/sealant layer.

<Measurement of oxygen permeation coefficient>

The oxygen permeation rate of the resultant laminate film X of Example 9 was measured by the aforementioned method. The results are shown in Table 4.

<Measurement of laminate strength>

Laminate film Y of Example 9 was prepared in the same conditions as those used in forming laminate film X of Example 9. The laminate strength of laminate film Y was measured by the aforementioned method. The results are shown in Table 4.

<Oxygen permeation rate of laminate film (ml/$m^2$·day·MPa) after flexing>

Using a Gelbo Flex tester (manufactured by RKC Instrument Inc.) laminate film Y of Example 9 was twisted (360 degrees) 50 times. After flexing, the laminate strength of laminate film Y was measured by using the aforementioned method. The results are shown in Table 4.

Example 10

The same procedure as in Example 9 was repeated except that the epoxy resin composition B was applied to the polyester layer surface in place of the epoxy resin composition A to obtain laminate films X and Y of Example 10.

The oxygen permeation rate of laminate film X of Example 10, the laminate strength of laminate film Y, and the oxygen permeation rate of laminate film Y after flexing were measured in the same manner as in Example 9. The results

are shown in Table 4.

Example 11

The same procedure as in Example 9 was repeated except that the epoxy resin composition B was applied to the silica deposited surface of a deposited film and polyester layer surface in place of the epoxy resin composition A to obtain laminate films X and Y of Example 11.

The oxygen permeation rate of laminate film X of Example 11, the laminate strength of laminate film Y, and the oxygen permeation rate of laminate film Y after flexing were measured in the same manner as in Example 9. The results are shown in Table 4.

Example 12

The same procedure as in Example 11 was repeated except that a deposited film (manufactured by Mitsubishi Plastics, Inc. trade name: TECHBARRIER TXR) prepared by depositing silica onto a polyethylene terephthalate film of 12 μm in thickness having a coated layer on the deposition surface was used in place of the deposited film (manufactured by Mitsubishi Plastics, Inc. trade name: TECHBARRIER L) prepared by depositing silica onto a polyethylene terephthalate film of 12 μm in thickness to obtain laminate films X and Y of Example 12.

The oxygen permeation rate of laminate film X of Example 12, the laminate strength of laminate film Y and the oxygen permeation rate of laminate film Y after flexing were measured in the same manner as in Example 9. The results are shown in Table 4.

Example 13

The same procedure as in Example 11 was repeated except that a deposited film (manufactured by TORAY ADVANCED FILM Co., Ltd, trade name: barrierlocks 1011HG) prepared by depositing alumina onto a polyethylene terephthalate film of 12 μm in thickness was used in place of the deposited film (manufactured by Mitsubishi Plastics, Inc. trade name: TECHBARRIER L) prepared by depositing silica onto a polyethylene terephthalate film of 12 μm in thickness to obtain laminate films X and Y of Example 13.

The oxygen permeation rate of laminate film X of Example 13, the laminate strength of laminate film Y, and the oxygen permeation rate of laminate film Y after flexing were measured in the same manner as in Example 9. The results are shown in Table 4.

Example 14

The same procedure as in Example 11 was repeated except that a deposited film (manufactured by TOYOBO CO, LTD, trade name: ECO-CR VE100) prepared by depositing two elements of silica and alumina onto a polyethylene terephthalate film of 12 μm in thickness was used in place of the deposited film (manufactured by Mitsubishi Plastics, Inc., trade name: TECHBARRIER L) prepared by depositing silica to a polyethylene terephthalate film of 12 μm in thickness to obtain laminate films X and Y of Example 14.

The oxygen permeation rate of laminate film X of Example 14, the laminate strength of laminate film Y, and the oxygen permeation rate of laminate film Y after flexing were measured in the same manner as in Example 9. The results are shown in Table 4.

Example 15

The same procedure as in Example 11 was repeated except that a deposited film (manufactured by Mitsubishi Plastics, Inc. trade name: TECHBARRIER NY) prepared by depositing silica onto a drawn 6-nylon film of 15 μm in thickness was used in place of the deposited film (manufactured by Mitsubishi Plastics, Inc. trade name: TECHBARRIER L) prepared by depositing silica onto a polyethylene terephthalate film of 12 μm in thickness to obtain laminate films X and Y of Example 15.

The oxygen permeation rate of laminate film X of Example 15, the laminate strength of laminate film Y, and the oxygen permeation rate of laminate film Y after flexing were measured in the same manner as in Example 9. The results are shown in Table 4.

Example 16

Onto the drawn polyester film (manufactured by Toyobo Co., Ltd., trade name :E5100) of 12 μm in thickness

serving as an outer layer, the epoxy resin composition B as mentioned above was applied by a gravure roll (100 lines/inch, depth: 100 μm), dried in a dry oven having a temperature of 60°C (near inlet) to 90°C (near outlet). Thereafter, the coated surface coated with the epoxy resin composition B was bonded to the silica deposited surface of the deposited film (manufactured by Mitsubishi Plastics, Inc. trade name: TECHBARRIER L), which was prepared by depositing silica onto a polyethylene terephthalate film of 12 μm in thickness, by a nip roll heated to 70°C, rolled up at a roll-up speed of 130 m/min. The roll was subjected to aging at 40°C for 2 days to obtain a laminate film consisting of outer layer/gas-barrier adhesive layer/vapor deposition layer/a base material.

Subsequently, to the surface of the base material of the obtained laminate film, the epoxy resin composition B was applied by a gravure roll (100 lines/inch, depth 100 μm) and subsequently dried in a dry oven having a temperature of 60°C (near inlet) to 90°C (near outlet). Thereafter, a low-density linear polyethylene film (manufactured by Mitsui Chemicals Tohcello Inc., trade name: TUX-MCS) of 40 μm in thickness was bonded thereto by a nip roll heated to 70°C, rolled up at a roll-up speed of 130 m/min. The roll was subjected to aging at 40°C for 2 days to obtain laminate films X and Y of Example 16 consisting of outer layer/gas-barrier adhesive layer/deposited layer/base material /gas-barrier adhesive layer/sealant layer.

The oxygen permeation rate of laminate film X of Example 16, the laminate strength of laminate film Y, and the oxygen permeation rate of laminate film Y after flexing were measured in the same manner as in Example 9. The results are shown in Table 4.

Example 17

Onto the drawn polyester film (manufactured by Toyobo Co., Ltd., trade name :E5100) of 12 μm in thickness serving as an outer layer, the epoxy resin composition B as mentioned above was applied by a gravure roll (100 lines/inch, depth: 100 μm), dried in a dry oven having a temperature of 60°C (near inlet) to 90°C (near outlet). Thereafter, the coated surface coated with the epoxy resin composition B was bonded to the polyethylene terephthalate film surface (manufactured by Mitsubishi Plastics, Inc. trade name: TECHBARRIER L), which was prepared by depositing silica onto a polyethylene terephthalate film of 12 μm in thickness, by a nip roll heated to 70°C, rolled up at a roll-up speed of 130 m/min. The roll was subjected to aging at 40°C for 2 days to obtain a laminate film consisting of outer layer/gas-barrier adhesive layer/a base material/vapor deposition layer.

Subsequently, to the surface of the vapor deposition layer of the obtained laminate film, the epoxy resin composition B was applied by a gravure roll (100 lines/inch, depth 100 μm) and subsequently dried in a dry oven having a temperature of 60°C (near inlet) to 90°C (near outlet). Thereafter, the coated surface coated with the epoxy resin composition B and a low-density linear polyethylene film (manufactured by Mitsui Chemicals Tohcello Inc., trade name: TUX-MCS) of 40 μm in thickness were bonded by a nip roll heated to 70°C, rolled up at a roll-up speed of 130 m/min. The roll was subjected to aging at 40°C for 2 days to obtain laminate films X and Y of Example 17 consisting of outer layer/gas-barrier adhesive layer/base material/deposited layer/gas-barrier adhesive layer/sealant layer.

The oxygen permeation rate of laminate film X of Example 17, the laminate strength of laminate film Y, and the oxygen permeation rate of laminate film Y after flexing were measured in the same manner as in Example 9. The results are shown in Table 4.

Example 18

The same procedure as in Example 11 was repeated except that an ethyl acetate solution (solid-content concentration: 30% by mass) containing a polyether component (manufactured by Toyo-Morton, Ltd.; TM-319) (50 parts by mass) and a polyisocyanate component (manufactured by Toyo-Morton, Ltd. CAT-19B)(50 parts by mass) was applied as a polyurethane adhesive in place of the epoxy resin composition B to a polyester layer and dried at 85°C for 10 seconds, to prepare laminate films X and Y of Example 18.

The oxygen permeation rate of laminate film X of Example 18, the laminate strength of laminate film Y, and the oxygen permeation rate of laminate film Y after flexing were measured in the same manner as in Example 9. The results are shown in Table 4.

[Table 4]

| | Laminate strength of deposited film (g/15 mm) | Oxygen permeation rate of deposited film (ml/m$^2$·day·MPa) | Oxygen permeation rate of deposited film after 50 times flection (ml/m$^2$·day·MPa) |
|---|---|---|---|
| Example 9 | 220 | 1.5 | 18.6 |

(continued)

| | Laminate strength of deposited film (g/15 mm) | Oxygen permeation rate of deposited film (ml/m$^2$·day·MPa) | Oxygen permeation rate of deposited film after 50 times flection (ml/m$^2$·day·MPa) |
|---|---|---|---|
| Example 10 | 240 | 1.4 | 18.0 |
| Example 11 | 230 | 1.2 | 17.3 |
| Example 12 | 250 | 1.1 | 19.1 |
| Example 13 | 230 | 2.2 | 23.0 |
| Example 14 | 240 | 3.0 | 19.2 |
| Example 15 | 260 | 1.3 | 17.4 |
| Example 16 | 230 | 1.2 | 18.3 |
| Example 17 | 230 | 1.4 | 17.6 |
| Example 18 | 220 | 1.2 | 19.2 |

Example 19

A silica deposited polyester film (manufactured by Mitsubishi Plastics, Inc., trade name: TECHBARRIER L) of 12 μm in thickness was used as a base material and the epoxy resin composition A was applied (application quantity: 1.0 g/m$^2$ (solid content)) onto the deposited layer of the silica deposited polyester film by using a roll having a depth of 26 μm, and dried in a dry oven of 70°C. Subsequently, to gravure ink (manufactured by Dainichiseika Color & Chemicals, Mfg. Co, Ltd., trade name: NT-HiLamic-701R white containing 5% ofNT-HiLamic hardener, manufactured by Dainichiseika Color & Chemicals, Mfg. Co, Ltd.), a solvent mixture of ethyl acetate/MEK/IPA = 4/4/2 was added to prepare coating liquid A having a Zahn cup (No. 3) viscosity of 16 seconds (25°C). Thereafter, to the coated surface of the epoxy resin composition A, coating liquid A was applied by using a roll of 26 μm in depth, dried in a dry oven of 70°C, and then rolled up at a roll-up speed of 100 m/min to obtain gas-barrier film X (gas-barrier laminate) consisting of a base material/deposited layer/gas-barrier adhesive layer/print layer.

Subsequently, using a gravure roll (140 lines/inch, a depth of 75 μm), the epoxy resin composition A was applied (application quantity: 2.5 g/m$^2$ (solid content)) onto on the print layer, and then dried in a dry oven having 60°C (near inlet) to 90°C (near outlet). Thereafter, a drawn polyester film (manufactured by TOYOBO CO, LTD, trade name: E5200) of 12 μm in thickness was bonded thereto by a nip roll heated to 70°C and rolled up at a roll-up speed of 130 m/min. The roll was subjected to aging at 40°C for 2 days to obtain a gas barrier film (gas-barrier laminate) consisting of base material/deposited layer/gas-barrier adhesive layer/print layer/gas-barrier adhesive layer/PET base material.

Thereafter, using a gravure roll (140 lines/inch, a depth of 75 μm), the epoxy resin composition A was applied (application quantity: 2.5 g/m$^2$ (solid content)) onto PET base material, and then dried in a dry oven having 60°C (near inlet) to 90°C (near outlet). Thereafter, a low-density linear polyethylene film (manufactured by Mitsui Chemicals Tohcello Inc., trade name: TUX-MCS) of 40 μm in thickness was bonded thereto by a nip roll heated to 70°C and rolled up at a roll-up speed of 120 m/min. The roll was subjected to aging at 40°C for 2 days to prepare laminate film Y consisting of a base material/deposited layer/gas-barrier adhesive layer/print layer/gas-barrier adhesive layer/PET base material/gas-barrier adhesive layer/sealant layer.

The oxygen permeation rates of laminate film X, deposited film, and print layer of Example 19 were separately measured under the aforementioned conditions. The oxygen permeation coefficient of the gas barrier adhesive layer formed of a cured product of the epoxy resin composition A was calculated in accordance with the aforementioned formula for computation (in the above formula, $R_1$ represents the oxygen permeation rate of laminate film X, $R_2$ represents

the oxygen permeation rate of the deposited film, $R_3$ represents the oxygen permeation rate of the print layer). The results are shown in Table 5.

Furthermore, the laminate strength and oxygen permeation rate of laminate film Y of Example 19 were measured by the aforementioned method. The results are shown in Table 5.

Furthermore, using a Gelbo Flex tester (manufactured by RKC Instrument Inc.), laminate film Y of Example 19 was twisted (360 degrees) 50 times. After flexing, the laminate strength of laminate film Y was measured by using the aforementioned method. The results are shown in Table 5.

Example 20

The same procedure as in Example 19 was repeated except that the epoxy resin composition B was used in place of the epoxy resin composition A to obtain laminate films X and Y of Example 20.

The oxygen permeation rate of laminate film X of Example 20, the laminate strength of laminate film Y of Example 20, and the oxygen permeation rate of laminate film Y after flexing were measured in the same manner as in Example 19. The results are shown in Table 5.

Example 21

The same procedure as in Example 19 was repeated except that a deposited film (manufactured by TORAY ADVANCED FILM Co., Ltd, trade name: barrierlocks 1011HG) prepared by depositing alumina onto a polyethylene terephthalate film of 12 μm in thickness was used in place of the silica deposited polyester film (manufactured by Mitsubishi Plastics, Inc. trade name: TECHBARRIER L) of 12 μm in thickness to obtain laminate films X and Y of Example 21.

The oxygen permeation coefficient of laminate film X of Example 21 and the laminate strength and the oxygen permeation rate of laminate film Y of Example 21, and the oxygen permeation rate of laminate film Y after flexing were measured in the same manner as in Example 19. The results are shown in Table 5.

Example 22

The same procedure as in Example 19 was repeated except that a deposited film (manufactured by TOYOBO CO, LTD, trade name: ECO-CR VE100) prepared by depositing two elements of silica and alumina onto a polyethylene terephthalate film of 12 μm in thickness was used in place of the deposited film (manufactured by Mitsubishi Plastics, Inc., trade name: TECHBARRIER L) prepared by depositing silica to a polyester film of 12 μm in thickness to obtain laminate films X and Y of Example 22.

The oxygen permeation coefficient of laminate film X of Example 22 and the laminate strength of laminate film Y of Example 22 were measured in the same manner as in Example 19. The results are shown in Table 5.

Example 23

The same procedure as in Example 19 was repeated except that a deposited film (manufactured by Mitsubishi Plastics, Inc., trade name: TECHBARRIER NR) prepared by depositing silica onto a nylon film of 15 μm in thickness was used in place of the deposited film (manufactured by Mitsubishi Plastics, Inc., trade name: TECHBARRIER L) prepared by depositing silica to obtain laminate films X and Y of Example 23.

The oxygen permeation coefficient of laminate film X of Example 23 and the laminate strength and the oxygen permeation rate of laminate film Y of Example 23, and the oxygen permeation rate of laminate film Y after flexing were measured in the same manner as in Example 19. The results are shown in Table 5.

Example 24

The same procedure as in Example 19 was repeated except that an ethyl acetate solution (solid-content concentration: 30% by mass) containing a polyether component (manufactured by Toyo-Morton, Ltd.; TM-319) (50 parts by mass) and a polyisocyanate component (manufactured by Toyo-Morton, Ltd. CAT-19B)(50 parts by mass) was applied as a polyurethane adhesive in place of the epoxy resin composition A to a print layer and a PET base material to prepare laminate films X and Y of Example 24.

The oxygen permeation rate of laminate film X of Example 24, the laminate strength and the oxygen permeation rate of laminate film Y of Example 24, and the oxygen permeation rate of laminate film Y after flexing were measured in the same manner as in Example 19. The results are shown in Table 5.

Example 25

The same procedure as in Example 20 was repeated except that an ethyl acetate solution (solid-content concentration: 30% by mass) containing a polyether component (manufactured by Toyo-Morton, Ltd.; TM-319) (50 parts by mass) and a polyisocyanate component (manufactured by Toyo-Morton, Ltd. CAT-19B)(50 parts by mass) was applied as a polyurethane adhesive in place of the epoxy resin composition B to a print layer and a PET base material to prepare laminate films X and Y of Example 25.

The oxygen permeation rate of laminate film X of Example 25, the laminate strength and the oxygen permeation rate of laminate film Y of Example 25, and the oxygen permeation rate of laminate film Y after flexing were measured in the same manner as in Example 19. The results are shown in Table 5.

[Table 5]

| | Laminate strength (g/15 mm) | Oxygen permeation coefficient of gas-barrier adhesive layer (ml·mm/m$^2$·day·MPa) | Oxygen permeation rate before flexing (ml/m$^2$·day·MPa) | Oxygen permeation rate after flexing 50-times (ml/m$^2$·day·MPa) |
|---|---|---|---|---|
| Example 19 | 250 | 0.89 | 1.3 | 18.1 |
| Example 20 | 230 | 0.93 | 1.0 | 17.8 |
| Example 21 | 220 | 0.93 | 2.0 | 21.5 |
| Example 22 | 240 | 0.93 | 2.7 | 19.7 |
| Example 23 | 260 | 0.93 | 1.1 | 18.5 |
| Example 24 | 300 | 0.93 | 1.5 | 19.0 |
| Example 25 | 280 | 0.93 | 1.6 | 19.1 |

Example 26

An ethyl acetate solution (solid-content concentration: 30% by mass) containing a polyether component ((manufactured by Toyo-Morton, Ltd.; trade name: TM-319) (50 parts by mass) and a polyisocyanate component (manufactured by Toyo-Morton, Ltd. trade name:CAT-19B)(50 parts by mass) was applied (application quantity: 3.5 g/m2 (solid content)) to a biaxially drawn nylon film (manufactured by TOYOBO CO, LTD; N1102) of 15 μm in thickness by using bar coater No. 6, dried at 85°C for 10 seconds. Thereafter, a drawn polyester film (manufactured by TOYOBO CO, LTD, trade name: E5200) of 12 μm in thickness was bonded thereto by a nip roll and subjected to aging at 40°C for 2 days to obtain a laminate.

Using bar coater No. 8, the epoxy resin composition A was applied (application quantity: 3.5 g/m$^2$ (solid content)) onto the polyester film of the obtained laminate, and dried at 85°C for 10 seconds. Thereafter, a low-density linear polyethylene film (manufactured by Mitsui Chemicals Tohcello Inc., trade name: TUX-MCS) of 40 μm in thickness was bonded thereto by a nip roll and subjected to aging at 40°C for 2 days to obtain the laminate film of Example 26.

Subsequently, two laminate films of Example 26 were prepared and laminated such that low-density linear polyethylene film surfaces faced each other. The three edges of the outer peripheral thereof were sealed with heat to form sealing portions. In this manner, a packaging bag with a top opened and three sides sealed was prepared. Into the packaging bag thus prepared, limonene (2 g) was enclosed from the opening portion. Thereafter, the packaging bag was placed in a glass container and the container was sealed hermetically.

Subsequently, the oxygen permeation rate and laminate strength of the laminate film of Example 26 were measured by the aforementioned method. Furthermore, measurement of laminate strength and odor retention test were carried out. The results are shown in Table 6.

Note that laminate strength measurement and evaluation in odor retention test are as follows.

<Laminate strength (g/15 mm)>

In accordance with the method defined in JIS K-6854, the laminate strength of a laminate film was measured by a T-peel test at a peel speed of 300 mm/min. In this case, the laminate strength was measured before a content was stored (initial) and after storage of the content for 14 days.

<Odor retention test>

Bags having a size of 5 cm x 5 cm were prepared from laminate films. A content (2 g) was sealed hermetically in each of the bags, stored hermetically in a glass container at a temperature of 23°C and a relative humidity of 60% for 1 to 14 days. The presence or absence of odor leakage was evaluated by a sensory test every hour. A bag retaining odor of the content was evaluated as ○, a bag from which a little leakage of odor was detected was evaluated as Δ, and a laminate film from which leakage of odor is clearly detected was evaluated as x.

Examples 27 to 31

A laminate film and packaging bag of Examples 27 to 31 were prepared in the same manner as in Example 26 except that the epoxy resin composition B was used in place of the epoxy resin composition A.
To packaging bags obtained in Examples 27 to 31, limonene (2 g), methyl salicylate (2 g), p-dichlorobenzene (2 g), curry powder (2 g) and clove (2 g) were separately enclosed and sealed hermetically and these bags were placed hermetically in a glass container.
Subsequently, the oxygen permeation rate and laminate strength of the laminate film of Examples 27 to 31 were measured and odor retention test was carried out in the same manner as in Example 26. The results are shown in Table 6.

[Table 6]

| | Content | Oxygen permeation rate | Laminate strength | | Odor retention properties | | | |
|---|---|---|---|---|---|---|---|---|
| | | ml/m$^2$·day·MPa | Initial | 14 days later | 1 | 3 | 7 | 14 |
| Example 26 | Limonene | 135 | 250 | 230 | ○ | ○ | ○ | ○ |
| Example 27 | Limonene | 140 | 220 | 210 | ○ | ○ | ○ | ○ |
| Example 28 | Methyl salicylate | 138 | 230 | 250 | ○ | ○ | ○ | ○ |
| Example 29 | p-Dichloro benzene | 141 | 260 | 240 | ○ | ○ | ○ | ○ |
| Example 30 | Curry powder | 139 | 240 | 250 | ○ | ○ | ○ | ○ |
| Example 31 | Clove | 137 | 250 | 220 | ○ | ○ | ○ | ○ |

As is described in the foregoing, the epoxy resin curing agent, the epoxy resin composition and the gas-barrier laminate of the present invention can express high gas-barrier properties and excellent adhesiveness to various types of plastics, in particular, to polyester, at the same time, and are thus widely and effectively available in the fields including packaging materials for food and medicines requiring gas-barrier properties and for coatings requiring corrosion proofing and gas-barrier properties, and used particularly in gas-barrier adhesives for a plastic film such as polyolefin, polyester and polyamide used as a packaging material for e.g., food and medicines, or a gas-barrier laminate.

## Claims

1. An epoxy resin curing agent **characterized by** being a reaction product of the following (A) and (B) only:

(A) metaxylylenediamine or paraxylylenediamine;
(B) an unsaturated carboxylic acid represented by the following formula (1) and/or derivatives thereof:

[Formula 1]

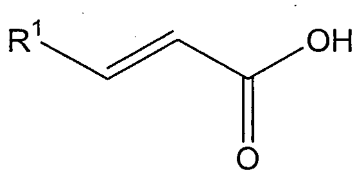

(1)

wherein $R^1$ represents an alkyl group having 1 to 8 carbon atoms, an aralkyl group having 1 to 8 carbon atoms or an aryl group.

2. An epoxy resin curing agent **characterized by** being a reaction product between the following (A), (B) and further at least one compound selected from the group consisting of the following (C), (D) and (E):

(A) metaxylylenediamine or paraxylylenediamine;
(B) an unsaturated carboxylic acid represented by the following formula (1) and/or derivatives thereof:

[Formula 2]

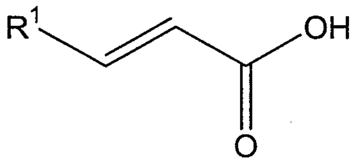

(1)

wherein $R^1$ represents an alkyl group having 1 to 8 carbon atoms, an aralkyl group having 1 to 8 carbon atoms or an aryl group;
(C) monovalent carboxylic acids represented by $R^2$-COOH and/or derivatives thereof, wherein $R^2$ represents a hydrogen atom, an alkyl group having 1 to 7 carbon atoms which may have a hydroxyl group, or an aryl group;
(D) a cyclic carbonate; and
(E) a monoepoxy compound having 2 to 20 carbon atoms.

3. The epoxy resin curing agent according to claim 1 or 2, wherein the (A) component is metaxylylene diamine.

4. The epoxy resin curing agent according to any one of claims 1 to 3, wherein the unsaturated carboxylic acid derivatives of the (B) component represented by the formula (1) are esters, amides, acid anhydrides or acid chlorides.

5. The epoxy resin curing agent according to any one of claims 1 to 4, wherein the (B) component is at least one selected from the group consisting of crotonic acid and crotonic acid esters.

6. The epoxy resin curing agent according to any one of claims 2 to 5, wherein the (C) component is at least one selected from the group consisting of formic acid, acetic acid, propionic acid, butyric acid, lactic acid, glycolic acid, benzoic acid and derivatives of these.

7. The epoxy resin curing agent according to any one of claims 2 to 6, wherein the (D) component is at least one selected from the group consisting of ethylene carbonate, propylene carbonate and glycerin carbonate.

8. The epoxy resin curing agent according to any one of claims 2 to 7, wherein the (E) component is a compound represented by the following formula (2):

[Formula 3]

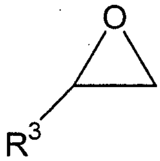

(2)

wherein $R^3$ represents a hydrogen atom, an alkyl group having 1 to 8 carbon atoms, an aryl group, or $R^4$-O-$CH_2$ and $R^4$ represents a phenyl group or a benzyl group.

**9.** The epoxy resin curing agent according to any one of claims 1 to 8, wherein a reaction molar ratio of the (B) component to the (A) component is in a range of 0.3 to 1.0.

**10.** An epoxy resin composition at least comprising an epoxy resin and the epoxy resin curing agent according to any one of claims 1 to 9.

**11.** The epoxy resin composition according to claim 10, wherein a ratio of the number of active amine hydrogen in the epoxy resin curing agent to the number of epoxy groups in the epoxy resin is in a range of 0.2 to 12.0.

**12.** The epoxy resin composition according to claim 10 or 11, wherein the epoxy resin is at least one resin selected from the group consisting of an epoxy resin derived from metaxylylene diamine and having a glycidylamino group, an epoxy resin derived from 1,3-bis(aminomethyl)cyclohexane and having a glycidylamino group, an epoxy resin derived from diaminodiphenylmethane and having a glycidylamino group, an epoxy resin derived from paraaminophenol and having a glycidylamino group and/or a glycidyloxy group, an epoxy resin derived from bisphenol A and having a glycidyloxy group, an epoxy resin derived from bisphenol F and having a glycidyloxy group, an epoxy resin derived from phenolnovolac and having a glycidyloxy group, and an epoxy resin derived from resorcinol and having a glycidyloxy group.

**13.** The epoxy resin composition according to claim 12, wherein the epoxy resin comprises the epoxy resin derived from metaxylylene diamine and having a glycidylamino group and/or the epoxy resin derived from bisphenol F and having a glycidyloxy group, as a main component(s).

**14.** The epoxy resin composition according to claim 13, wherein the epoxy resin comprises the epoxy resin derived from metaxylylene diamine and having a glycidylamino group, as a main component.

**15.** A gas-barrier adhesive comprising the epoxy resin composition according to any one of claims 10 to 14.

**16.** A gas-barrier laminate at least comprising a flexible polymer film layer, a paper layer and/or a metal foil layer and at least one gas barrier layer, wherein the gas barrier layer is a layer formed by curing the epoxy resin composition according to any one of claims 10 to 14.

**17.** A gas-barrier container obtained by molding a gas-barrier laminate sheet at least having at least one flexible polymer layer and at least one gas barrier adhesive layer, wherein the gas barrier adhesive layer is formed by curing the epoxy resin composition according to any one of claims 10 to 14.

**18.** A deposited film formed by at least laminating a base material, at least one deposited layer selected from the group consisting of a silica deposited layer, an alumina deposited layer and a silica-alumina two-element deposited layer, a gas-barrier adhesive layer and a sealant layer, wherein the gas barrier adhesive layer is formed by curing the epoxy resin composition according to any one of claims 10 to 14.

**19.** A gas-barrier laminate obtained by at least laminating a base material, a gas-barrier adhesive layer and a print layer, wherein the gas barrier adhesive layer is formed by curing the epoxy resin composition according to any one of claims 10 to 14.

**20.** A method for storing a content hermetically by placing a content within a packaging material using a gas-barrier laminate having at least one gas-barrier adhesive layer formed by curing the epoxy resin composition according to any one of claims 10 to 14.

## Patentansprüche

**1.** Epoxidharzhärtungsmittel, **dadurch gekennzeichnet, dass** es ein Reaktionsprodukt von ausschließlich den folgenden (A) und (B) ist:

(A) Metaxylylendiamin oder Paraxylylendiamin;

(B) eine ungesättigte Carbonsäure, dargestellt durch die folgende Formel (1), und/oder Derivate davon:

[Formel 1]

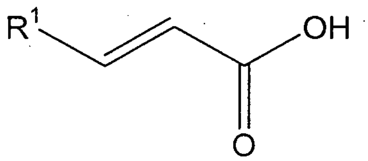

(1)

wobei $R^1$ eine Alkylgruppe mit 1 bis 8 Kohlenstoffatomen, eine Aralkylgruppe mit 1 bis 8 Kohlenstoffatomen oder eine Arylgruppe darstellt.

2. Epoxidharzhärtungsmittel, **dadurch gekennzeichnet, dass** es ein Reaktionsprodukt der folgenden (A), (B) und weiter mindestens einer Verbindung, ausgewählt aus der Gruppe, bestehend aus den folgenden (C), (D) und (E), ist:

(A) Metaxylylendiamin oder Paraxylylendiamin;
(B) eine ungesättigte Carbonsäure, dargestellt durch die folgende Formel (1), und/oder Derivate davon:

[Formel 2]

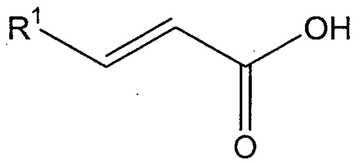

(1)

wobei $R^1$ eine Alkylgruppe mit 1 bis 8 Kohlenstoffatomen, eine Aralkylgruppe mit 1 bis 8 Kohlenstoffatomen oder eine Arylgruppe darstellt;
(C) einwertige Carbonsäuren, dargestellt durch $R^2$-COOH, und/oder Derivate davon, wobei $R^2$ ein Wasserstoffatom, eine Alkylgruppe mit 1 bis 7 Kohlenstoffatomen, die eine Hydroxylgruppe aufweisen kann, oder eine Arylgruppe darstellt;
(D) ein zyklisches Carbonat; und
(E) eine Monoepoxyverbindung mit 2 bis 20 Kohlenstoffatomen.

3. Epoxidharzhärtungsmittel nach Anspruch 1 oder 2, wobei der Bestandteil (A) Metaxylylendiamin ist.

4. Epoxidharzhärtungsmittel nach einem der Ansprüche 1 bis 3, wobei die ungesättigten Carbonsäurederivate des Bestandteils (B), dargestellt durch die Formel (1), Ester, Amide, Säureanhydride oder Säurechloride sind.

5. Epoxidharzhärtungsmittel nach einem der Ansprüche 1 bis 4, wobei der Bestandteil (B) mindestens eines, ausgewählt aus der Gruppe, bestehend aus Crotonsäure und Crotonsäureestern, ist.

6. Epoxidharzhärtungsmittel nach einem der Ansprüche 2 bis 5, wobei der Bestandteil (C) mindestens eines, ausgewählt aus der Gruppe, bestehend aus Ameisensäure, Essigsäure, Propionsäure, Buttersäure, Milchsäure, Glycolsäure, Benzoesäure und Derivaten von diesen, ist.

7. Epoxidharzhärtungsmittel nach einem der Ansprüche 2 bis 6, wobei der Bestandteil (D) mindestens eines, ausgewählt aus der Gruppe, bestehend aus Ethylencarbonat, Propylencarbonat und Glycerincarbonat, ist.

8. Epoxidharzhärtungsmittel nach einem der Ansprüche 2 bis 7, wobei der Bestandteil (E) eine Verbindung, dargestellt durch die folgende Formel (2), ist:

[Formel 3]

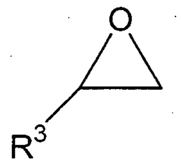

(2)

wobei $R^3$ ein Wasserstoffatom, eine Alkylgruppe mit 1 bis 8 Kohlenstoffatomen, eine Arylgruppe oder $R^4$-O-$CH_2$-darstellt und $R^4$ eine Phenylgruppe oder eine Benzylgruppe darstellt.

**9.** Epoxidharzhärtungsmittel nach einem der Ansprüche 1 bis 8, wobei ein Reaktionsmolverhältnis von dem Bestandteil (B) zu dem Bestandteil (A) in einem Bereich von 0,3 bis 1,0 liegt.

**10.** Epoxidharzzusammensetzung, mindestens umfassend ein Epoxidharz und das Epoxidharzhärtungsmittel nach einem der Ansprüche 1 bis 9.

**11.** Epoxidharzzusammensetzung nach Anspruch 10, wobei ein Verhältnis von der Anzahl von aktivem Aminwasserstoff in dem Epoxidharzhärtungsmittel zu der Anzahl von Epoxidgruppen in dem Epoxidharz in einem Bereich von 0,2 bis 12,0 liegt.

**12.** Epoxidharzzusammensetzung nach Anspruch 10 oder 11, wobei das Epoxidharz mindestens ein Harz ist, ausgewählt aus der Gruppe, bestehend aus einem Epoxidharz, welches von Metaxylylendiamin hergeleitet ist und eine Glycidylaminogruppe aufweist, einem Epoxidharz, welches von 1,3-Bis(aminomethyl)cyclohexan hergeleitet ist und eine Glycidylaminogruppe aufweist, einem Epoxidharz, welches von Diaminodiphenylmethan hergeleitet ist und eine Glycidylaminogruppe aufweist, einem Epoxidharz, welches von Paraaminophenol hergeleitet ist und eine Glycidylaminogruppe und/oder eine Glycidyloxygruppe aufweist, einem Epoxidharz, welches von Bisphenol A hergeleitet ist und eine Glycidyloxygruppe aufweist, einem Epoxidharz, welches von Bisphenol F hergeleitet ist und eine Glycidyloxygruppe aufweist, einem Epoxidharz, welches von Phenolnovolak hergeleitet ist und eine Glycidyloxygruppe aufweist, und einem Epoxidharz, welches von Resorcin hergeleitet ist und eine Glycidyloxygruppe aufweist.

**13.** Epoxidharzzusammensetzung nach Anspruch 12, wobei das Epoxidharz das Epoxidharz, welches von Metaxylylendiamin hergeleitet ist und eine Glycidylaminogruppe aufweist, und/oder das Epoxidharz, welches von Bisphenol F hergeleitet ist und eine Glycidyloxygruppe aufweist, als einen Hauptbestandteil(e) umfasst.

**14.** Epoxidharzzusammensetzung nach Anspruch 13, wobei das Epoxidharz das Epoxidharz, welches von Metaxylylendiamin hergeleitet ist und eine Glycidylaminogruppe aufweist, als einen Hauptbestandteil umfasst.

**15.** Gasbarrierenhaftmittel, umfassend die Epoxidharzzusammensetzung nach einem der Ansprüche 10 bis 14.

**16.** Gasbarrierenlaminat, mindestens umfassend eine flexible Polymerfilmschicht, eine Papierschicht und/oder eine Metallfolienschicht und mindestens eine Gasbarrierenschicht, wobei die Gasbarrierenschicht eine Schicht, gebildet durch Härten der Epoxidharzzusammensetzung nach einem der Ansprüche 10 bis 14, ist.

**17.** Gasbarrierenbehälter, erhalten durch Formen einer Gasbarrierenlaminatfolie, zumindest aufweisend mindestens eine flexible Polymerschicht und mindestens eine Gasbarrierenhaftmittelschicht, wobei die Gasbarrierenhaftmittelschicht durch Härten der Epoxidharzzusammensetzung nach einem der Ansprüche 10 bis 14 gebildet ist.

**18.** Abgeschiedene Folie, gebildet durch mindestens Laminieren eines Grundmaterials, mindestens einer abgeschiedenen Schicht, ausgewählt aus der Gruppe, bestehend aus einer abgeschiedenen Siliciumdioxidschicht, einer abgeschiedenen Aluminiumoxidschicht und einer abgeschiedenen Silicium-dioxid-Aluminiumoxid zwei-Element-Schicht, einer Gasbarrierenhaftmittelschicht und einer Dichtmittelschicht, wobei die Gasbarrierenhaftmittelschicht durch Härten der Epoxidharzzusammensetzung nach einem der Ansprüche 10 bis 14 gebildet ist.

**19.** Gasbarrierenlaminat, erhalten durch mindestens Laminieren eines Grundmaterials, einer Gasbarrierenhaftmittelschicht und einer Druckschicht, wobei die Gasbarrierenhaftmittelschicht durch Härten der Epoxidharzzusammensetzung nach einem der Ansprüche 10 bis 14 gebildet ist.

**20.** Verfahren zur hermetischen Lagerung eines Inhalts durch Platzieren eines Inhalts innerhalb eines Verpackungsmaterials unter Verwendung eines Gasbarrierenlaminats mit mindestens einer Gasbarrierenhaftmittelschicht, gebildet durch Härten der Epoxidharzzusammensetzung nach einem der Ansprüche 10 bis 14.

## Revendications

**1.** Agent de durcissement de résine époxyde **caractérisé en ce qu'**il est un produit de réaction des (A) et (B) qui suivent seulement :

(A) méta-xylylènediamine ou para-xylylènediamine ;
(B) un acide carboxylique insaturé représenté par la formule (1) suivante et/ou leurs dérivés :

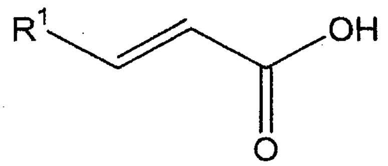

(1)

où $R^1$ représente un groupe alkyle ayant 1 à 8 atomes de carbone, un groupe aralkyle ayant 1 à 8 atomes de carbone ou un groupe aryle.

**2.** Agent de durcissement de résine époxyde **caractérisé en ce qu'**il est un produit de réaction des (A) et (B) qui suivent, et en outre, d'au moins un composé choisi parmi le groupe consistant des (C), (D) et (E) qui suivent :

(A) méta-xylylènediamine ou para-xylylènediamine ;
(B) un acide carboxylique insaturé représenté par la formule (1) suivante et/ou leurs dérivés :

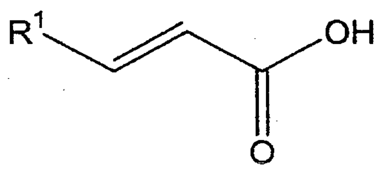

(1)

où $R^1$ représente un groupe alkyle ayant 1 à 8 atomes de carbone, un groupe aralkyle ayant 1 à 8 atomes de carbone ou un groupe aryle ;
(C) des acides carboxyliques monovalents représentés par $R^2$-COOH et/ou leurs dérivés, où $R^2$ représente l'atome d'hydrogène, un groupe alkyle ayant 1 à 7 atomes de carbone, qui peut porter un groupe hydroxyle, ou un groupe aryle ;
(D) un carbonate cyclique, et
(E) un composé monoépoxyde ayant 2 à 20 atomes de carbone.

**3.** Agent de durcissement de résine époxyde selon la revendication 1 ou 2, où le composant (A) est la méta-xylylènediamine.

**4.** Agent de durcissement de résine époxyde selon l'une quelconque des revendications 1 à 3, où les dérivés d'acide carboxylique insaturé du composant (B) représenté par la formule (1) sont des esters, des amides, des anhydrides d'acide ou des chlorures d'acide.

**5.** Agent de durcissement de résine époxyde selon l'une quelconque des revendications 1 à 4, où le composant (B) est au moins un choisi parmi le groupe consistant en de l'acide crotonique et des esters d'acide crotonique.

**6.** Agent de durcissement de résine époxyde selon l'une quelconque des revendications 2 à 5, où le composant (C) est au moins un choisi parmi le groupe consistant en l'acide formique, l'acide acétique, l'acide propionique, l'acide

butyrique, l'acide lactique, l'acide glycolique, l'acide benzoïque et leurs dérivés.

**7.** Agent de durcissement de résine époxyde selon l'une quelconque des revendications 2 à 6, où le composant (D) est au moins un choisi parmi le groupe consistant en le carbonate d'éthylène, le carbonate de propylène et le carbonate de glycérine.

**8.** Agent de durcissement de résine époxyde selon l'une quelconque des revendications 2 à 7, où le composant (E) est un composé représenté par la formule (2) suivante :

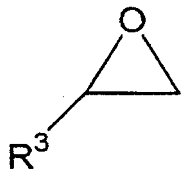

(2)

où $R^3$ représente l'atome d'hydrogène, un groupe alkyle ayant 1 à 8 atomes de carbone, un groupe aryle, ou $R^4$-O-$CH_2$- et $R^4$ représente un groupe phényle ou un groupe benzyle.

**9.** Agent de durcissement de résine époxyde selon l'une quelconque des revendications 1 à 8, où le rapport molaire du composant (B) au composant (A) dans la réaction se situe dans l'intervalle allant de 0,3 à 1,0.

**10.** Composition de résine époxyde comprenant au moins une résine époxyde et l'agent de durcissement de résine époxyde selon l'une quelconque des revendications 1 à 9.

**11.** Composition de résine époxyde selon la revendication 10, où le rapport du nombre d'hydrogène actif d'amine dans l'agent de durcissement de résine époxyde au nombre de groupes époxyde dans la résine époxyde se situe dans l'intervalle allant de 0,2 à 12,0.

**12.** Composition de résine époxyde selon la revendication 10 ou 11, où la résine époxyde est au moins une résine choisie parmi le groupe consistant en une résine époxyde dérivée de la méta-xylylènediamine et ayant un groupe glycidylamino, une résine époxyde dérivée du 1,3-bis(aminométhyl)cyclohexane et ayant un groupe glycidylamino, une résine époxyde dérivée du diaminodiphénylméthane et ayant un groupe glycidylamino, une résine époxyde dérivée du para-aminophénol et ayant un groupe glycidylamino et/ou un groupe glycidyloxy, une résine époxyde dérivée du bisphénol A et ayant un groupe glycidyloxy, une résine époxyde dérivée du bisphénol F et ayant un groupe glycidyloxy, une résine époxyde dérivée de la phénolnovolaque et ayant un groupe glycidyloxy, et une résine époxyde dérivée du résorcinol et ayant un groupe glycidyloxy.

**13.** Composition de résine époxyde selon la revendication 12, où la résine époxyde comprend la résine époxyde dérivée de la méta-xylylènediamine et ayant un groupe glycidylamino et/ou la résine époxyde dérivée du bisphénol F et ayant un groupe glycidyloxy, comme composants principaux.

**14.** Composition de résine époxyde selon la revendication 13, où la résine époxyde comprend la résine époxyde dérivée de la méta-xylylènediamine et ayant un groupe glycidylamino comme composant principal.

**15.** Adhésif de type barrière aux gaz comprenant la composition de résine époxyde selon l'une quelconque des revendications 10 à 14.

**16.** Stratifié de type barrière aux gaz comprenant au moins une couche flexible de film polymère, une couche en papier et/ou une couche de feuille métallique et au moins une couche de barrière aux gaz, où la couche de barrière aux gaz est une couche formée par durcissement de la composition de résine époxyde selon l'une quelconque des revendications 10 à 14.

**17.** Conteneur de type barrière aux gaz obtenu par moulage d'une feuille stratifiée de type barrière aux gaz comprenant au moins une couche flexible de film polymère et au moins une couche adhésive de barrière aux gaz, où la couche adhésive de barrière aux gaz est formée par durcissement de la composition de résine époxyde selon l'une quelconque des revendications 10 à 14.

**18.** Film déposé formé par stratification d'au moins un matériau de base, au moins une couche déposée choisie parmi une couche déposée de silice, une couche déposée d'alumine et une couche déposée à deux éléments silice-alumine, une couche adhésive de barrière aux gaz et une couche de scellement, où la couche adhésive de barrière aux gaz est formée par durcissement de la composition de résine époxyde selon l'une quelconque des revendications 10 à 14.

**19.** Stratifié de type barrière aux gaz obtenu par la stratification d'au moins un matériau de base, une couche adhésive de barrière aux gaz et une couche d'impression, où la couche adhésive de barrière aux gaz est formée par durcissement de la composition de résine époxyde selon l'une quelconque des revendications 10 à 14.

**20.** Procédé de stockage hermétique d'un contenu en plaçant le contenu dans un matériau d'emballage en utilisant un stratifié de type barrière aux gaz ayant au moins une couche adhésive de barrière aux gaz formée par durcissement de la composition de résine époxyde selon l'une quelconque des revendications 10 à 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7091367 B
- JP 7091368 B
- WO 09511537 A
- JP 2002256208 A
- JP 5051574 A
- JP 9316422 A
- JP 2000154365 A
- WO 9960068 A